(19) Europäisches Patentamt / European Patent Office / Office européen des brevets

(11) **EP 4 775 574 A1**

(12) **EUROPEAN PATENT APPLICATION**
published in accordance with Art. 153(4) EPC

(43) Date of publication:
**15.07.2026 Bulletin 2026/29**

(21) Application number: **24893458.0**

(22) Date of filing: **20.11.2024**

(51) International Patent Classification (IPC):
***C07D 405/12*** (2006.01) ***C07D 405/14*** (2006.01)
***C07D 413/14*** (2006.01) ***C07D 471/04*** (2006.01)
***C07D 471/08*** (2006.01) ***C07D 487/10*** (2006.01)
***C07D 491/048*** (2006.01) ***C07D 491/08*** (2006.01)
***C07D 491/10*** (2006.01) ***C07D 493/10*** (2006.01)
***C07D 498/04*** (2006.01) ***C07D 498/08*** (2006.01)
***A61K 31/443*** (2006.01) ***A61K 31/497*** (2006.01)
***A61P 29/00*** (2006.01)

(52) Cooperative Patent Classification (CPC):
**A61K 31/341; A61K 31/443; A61K 31/497; A61P 9/06; A61P 13/00; A61P 25/00; A61P 25/04; A61P 29/00; C07D 307/24; C07D 405/12; C07D 405/14; C07D 407/12; C07D 413/14; C07D 471/04; C07D 471/08;** (Cont.)

(86) International application number:
**PCT/CN2024/133203**

(87) International publication number:
**WO 2025/108301 (30.05.2025 Gazette 2025/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.11.2023 CN 202311557253**
**10.01.2024 CN 202410037487**

(71) Applicant: **Wuhan Xirui Pharmaceutical Technology Co., Ltd**
**Wuhan, Hubei 430000 (CN)**

(72) Inventors:
- **LI, Weiyong**
**Wuhan, Hubei 430000 (CN)**
- **HE, Min**
**Wuhan, Hubei 430000 (CN)**
- **ZHOU, Yuan**
**Wuhan, Hubei 430000 (CN)**
- **FU, Ruixin**
**Wuhan, Hubei 430000 (CN)**
- **HU, Hao**
**Wuhan, Hubei 430000 (CN)**
- **TIAN, Nian**
**Wuhan, Hubei 430000 (CN)**

(74) Representative: **Henderson, Helen Lee**
**Withers & Rogers LLP**
**2 London Bridge**
**London SE1 9RA (GB)**

(54) **FURAN RING-CONTAINING COMPOUND, PHARMACEUTICAL COMPOSITION THEREOF, AND USE THEREOF**

(57) The present invention relates to a furan ring-containing compound, a pharmaceutical composition thereof, and a use thereof. Specifically disclosed is a compound as represented by formula (I) or a pharmaceutically acceptable salt thereof. The compound of the present invention has one or more of the following effect advantages: (1) a novel structure; (2) a good blocking effect (inhibitory effect) on the activity of a Nav1.8 channel; and (3) good selectivity for other subtypes of Nav and high safety.

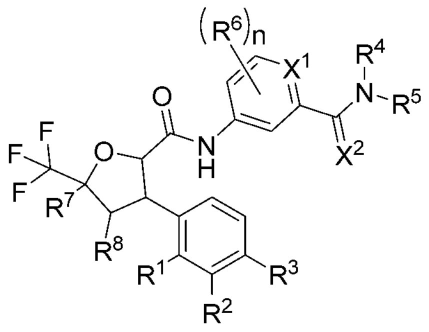

(I)

(52) Cooperative Patent Classification (CPC): (Cont.)
**C07D 487/10; C07D 491/048; C07D 491/08; C07D 491/10; C07D 493/10; C07D 498/04; C07D 498/08**

## Description

The present application claims priority to Chinese Patent Application No. 2023115572534 filed on November 21, 2023 and Chinese Patent Application No. 2024100374874 filed on January 10, 2024. The contents of the Chinese patent application are incorporated herein by reference in their entireties.

TECHNICAL FIELD

The present disclosure relates to a furan ring-containing compound, a pharmaceutical composition thereof, and a use thereof.

BACKGROUND

Pain originates from nociceptors in the peripheral nervous system. These are free nerve endings widely distributed throughout the skin, muscles, joints, and visceral tissues of the body. They can convert perceived thermal, mechanical, or chemical stimuli into nerve impulses (action potentials) and transmit them via afferent nerve fibers to their cell bodies located in the dorsal root ganglia (DRG), ultimately transmitting to higher neural centers and causing pain perception. The generation and conduction of action potentials in neurons depend on the voltage-gated sodium channels (VGSC / Nav) on the cell membrane, and Nav is the key mediator for transmembrane information transmission along neurons.

According to statistics, approximately one-fifth of the world's population suffers from moderate to severe chronic pain. In 2018, the global analgesic market was approximately 36 billion USD, and it is expected to reach 56 billion USD by 2023. Among them, acute moderate to severe pain will grow steadily at a compound annual growth rate of 2.5% in the future, while the chronic pain market will grow at a compound annual growth rate of approximately 18% in the future; chronic pain is the main driving force for the continuous growth of the global pain market over the next decade.

The secondary structure of voltage-gated sodium channels (VGSC / Nav) includes four domains (DI-DIV) forming the pore, each domain having six transmembrane $\alpha$-helices (S1-S6), among which the S4 segment is a voltage sensor containing positively charged ions; the intracellular loop between DIII and DIV is considered as the fast inactivation gate; Nav exists in three different states, and the inactivated closed state exhibits different kinetics of fast inactivation (within milliseconds) or slow inactivation (within seconds). When the cell membrane is depolarized, sodium channels are activated and the channels open, causing an influx of sodium ions, which further depolarizes the cell membrane and leads to the generation of an action potential. Therefore, inhibiting abnormal sodium channel activity helps in the treatment and alleviation of pain, and Nav is a potential peripheral target for pain therapy.

There are mainly nine subtypes of human Nav, namely Nav1.1-Nav1.9. According to whether they can be effectively inhibited by nanomolar tetrodotoxin (TTX), Nav is classified into TTX-sensitive (TTX-S) and TTX-resistant (TTX-R) types, and the tissue expression of different subtypes is extremely different. Nav1.1, Nav1.2, Nav1.3, Nav1.4, Nav1.6, and Nav1.7 are TTX-S types, among which Nav1.1, Nav1.2, and Nav1.3 are highly expressed in the central nervous system (CNS), Nav1.4 is abundantly present in skeletal muscle, and Nav1.6 and Nav1.7 are mainly abundantly present in the central nervous system. Nav1.5, Nav1.8, and Nav1.9 are TTX-R types, among which Nav1.5 is mainly present in cardiomyocytes, and Nav1.8 and Nav1.9 are present in the peripheral nervous system dorsal root ganglia (PNS-DRG).

Studies have shown that changes in ion channels are the molecular basis of peripheral sensitization, central sensitization, and disinhibition after inflammation or neuropathological injury, and are also the important molecular mechanism of pain occurrence. At present, Nav inhibitors have been proven to be effective, such as the local anesthetic lidocaine relieving pain by inhibiting Nav, and non-selective Nav inhibitors such as lamotrigine, lacosamide, and mexiletine have been successfully used in the treatment of chronic pain. However, the Nav inhibitors currently used in clinical practice lack subtype selectivity and can inhibit sodium channels expressed in the heart and central nervous system, resulting in a narrow therapeutic window and limited application range. Nav1.8 is an important ion channel involved in chronic pain, atrial fibrillation, and Budd-Chiari syndrome, and highly selective Nav1.8 inhibitors are ideal targets for pain treatment, with relatively fewer side effects when producing analgesic effects.

In models of neuropathic pain, nerve injury increases the expression level of Nav1.8 in axons and neuronal cell bodies. Using Nav1.8 antisense oligonucleotides can significantly alleviate pain while reducing the expression of Nav1.8. Nav1.8 knockout mice cannot exhibit normal visceral inflammatory pain. After the human Nav1.8 gene undergoes a gain-of-function mutation, it causes peripheral neuralgia. According to a series of animal experiments and human genetic evidence, selective inhibition of Nav1.8 has the potential to become a novel analgesic therapy that can be used for the treatment of various types of pain, including inflammatory pain, neuropathic pain, postoperative pain, and cancer pain.

Highly selective Nav inhibitors are one of the key research directions in the development of voltage-gated sodium channels. Since Nav1.8 is mainly distributed in the peripheral nervous system and confined to neurons that perceive pain,

it is a highly selective target for pain treatment. Therefore, selective inhibition of Nav1.8 shows good prospects for reducing potential toxic and side effects. The Nav inhibitors used in clinical practice lack subtype selectivity and can inhibit sodium channels expressed in the heart and central nervous system, resulting in a narrow therapeutic window and limited application range. Therefore, it is necessary to develop Nav1.8 inhibitors with higher activity, better selectivity, superior pharmacokinetic properties, and fewer side effects to meet clinical needs.

SUMMARY

The technical problem to be solved by the present disclosure is to overcome the deficiency of insufficient Nav1.8 channel inhibition in the prior art. To this end, the present disclosure provides a compound containing a furan ring, a pharmaceutical composition thereof, and a use thereof. The compounds of the present disclosure exhibit one or more of the following advantageous effects: (1) novel structure; (2) good blocking effect (inhibition) on Nav1.8 channel activity; (3) good selectivity toward other Nav subtypes (*e.g.*, Nav1.1, Nav1.2, Nav1.3, Nav1.4, Nav1.5, Nav1.6, Nav1.7, Nav1.9) with high safety.

The technical problem of the present disclosure is solved through the following technical solutions:

The present disclosure provides a compound of Formula I or a pharmaceutically acceptable salt thereof,

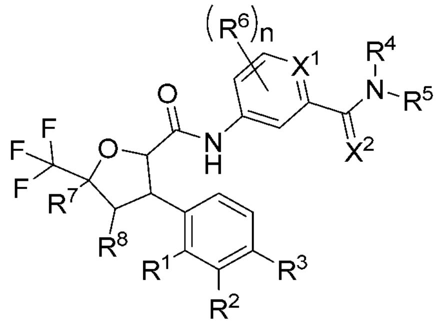

I ;

wherein $R^1$, $R^2$, and $R^3$ are each independently halogen, $C_1$-$C_6$ alkyl, $C_3$-$C_6$ cycloalkyl, $C_1$-$C_6$ alkoxy, -$OR^{1-1}$, $C_1$-$C_6$ alkyl substituted with one or more $R^{1-2}$, or $C_1$-$C_6$ alkoxy substituted with one or more $R^{1-3}$;

$R^{1-1}$ is $C_3$-$C_6$ cycloalkyl or 3- to 8-membered heterocycloalkyl; in the 3- to 8-membered heterocycloalkyl, the heteroatom is selected from 1, 2, or 3 types of N, O, and S, and the number of heteroatoms is 1, 2, or 3;

each $R^{1-2}$ is independently halogen;

each $R^{1-3}$ is independently $C_1$-$C_6$ alkoxy;

$X^1$ is N, $N^+$-$O^-$, or $CR^{X1}$;

$R^{X1}$ is hydrogen, halogen, or $C_1$-$C_6$ alkyl substituted with one or more halogens;

$X^2$ is O or NH;

$R^4$ is hydrogen or $C_1$-$C_6$ alkyl;

$R^5$ is $C_1$-$C_6$ alkoxy, -OH, -$NR^{5-1}R^{5-2}$, or -CN;

$R^{5-1}$ and $R^{5-2}$ are each independently hydrogen or $C_1$-$C_6$ alkyl;

n is 0, 1, 2, or 3;

$R^6$ is hydrogen, halogen, hydroxyl, $C_1$-$C_6$ alkyl, $C_1$-$C_6$ alkyl-$C_1$-$C_6$ alkoxy, or $C_1$-$C_6$ alkoxy;

$R^7$ is hydrogen or $C_1$-$C_6$ alkyl;

$R^8$ is hydrogen, hydroxyl, $C_1$-$C_6$ alkyl, $C_1$-$C_6$ alkyl-$C_1$-$C_6$ alkoxy, or $C_1$-$C_6$ alkoxy.

In some embodiments, in the compound of Formula I, $R^1$, $R^2$, and $R^3$ are each independently halogen, $C_1$-$C_6$ alkyl, $C_3$-$C_6$ cycloalkyl, $C_1$-$C_6$ alkoxy, -$OR^{1-1}$, $C_1$-$C_6$ alkyl substituted with one or more $R^{1-2}$, or $C_1$-$C_6$ alkoxy substituted with one or more $R^{1-3}$;

$R^{1-1}$ is $C_3$-$C_6$ cycloalkyl or 3- to 8-membered heterocycloalkyl; in the 3- to 8-membered heterocycloalkyl, the heteroatom is selected from 1, 2, or 3 types of N, O, and S, and the number of heteroatoms is 1, 2, or 3;

each $R^{1-2}$ is independently halogen;

each $R^{1-3}$ is independently $C_1$-$C_6$ alkoxy;

$X^1$ is N, $N^+$-$O^-$, or $CR^{X1}$;

$R^{X1}$ is hydrogen, halogen, or $C_1$-$C_6$ alkyl substituted with one or more halogens;

$X^2$ is O or NH;

$R^4$ is hydrogen or $C_1$-$C_6$ alkyl;

$R^5$ is $C_1$-$C_6$ alkoxy, -OH, or -$NR^{5-1}R^{5-2}$;
$R^{5-1}$ and $R^{5-2}$ are each independently hydrogen or $C_1$-$C_6$ alkyl;
n is 0, 1, 2, or 3;
$R^6$ is hydrogen, halogen, hydroxyl, $C_1$-$C_6$ alkyl, $C_1$-$C_6$ alkyl-$C_1$-$C_6$ alkoxy, or $C_1$-$C_6$ alkoxy;
$R^7$ is hydrogen or $C_1$-$C_6$ alkyl;
$R^8$ is hydrogen, hydroxyl, $C_1$-$C_6$ alkyl, $C_1$-$C_6$ alkyl-$C_1$-$C_6$ alkoxy, or $C_1$-$C_6$ alkoxy.

In some embodiments, in $R^1$, $R^2$, and $R^3$, each halogen is independently fluorine, chlorine, bromine, or iodine, preferably fluorine.

In some embodiments, in $R^1$, $R^2$, and $R^3$, each $C_1$-$C_6$ alkyl in the $C_1$-$C_6$ alkyl and the $C_1$-$C_6$ alkyl substituted with one or more $R^{1-2}$ is independently methyl, ethyl, *n*-propyl, isopropyl, *n*-butyl, isobutyl, or *tert*-butyl, preferably methyl.

In some embodiments, in $R^1$, $R^2$, and $R^3$, each $C_3$-$C_6$ cycloalkyl is independently cyclopropyl, cyclobutyl, cyclopentyl, or cyclohexyl, preferably cyclopropyl.

In some embodiments, in $R^1$, $R^2$, and $R^3$, each $C_1$-$C_6$ alkoxy in the $C_1$-$C_6$ alkoxy and the $C_1$-$C_6$ alkoxy substituted with one or more $R^{1-3}$ is independently methoxy, ethoxy, *n*-propoxy, isopropoxy, *n*-butoxy, isobutoxy, or *tert*-butoxy, preferably methoxy or ethoxy.

In some embodiments, in $R^{1-1}$, the $C_3$-$C_6$ cycloalkyl is cyclopropyl, cyclobutyl, cyclopentyl, or cyclohexyl, preferably cyclopropyl.

In some embodiments, in $R^{1-1}$, the heteroatom in the 3- to 8-membered heterocycloalkyl is N and/or O; the number of heteroatoms is preferably 1 or 2. The 3- to 8-membered heterocycloalkyl is preferably 4- to 6-membered heterocycloalkyl; more preferably

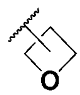 ;

for example,

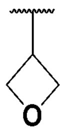 .

In some embodiments, in $R^{1-2}$, the halogen is fluorine, chlorine, bromine, or iodine, preferably fluorine.

In some embodiments, in $R^{1-3}$, the $C_1$-$C_6$ alkoxy is methoxy, ethoxy, *n*-propoxy, isopropoxy, *n*-butoxy, isobutoxy, or *tert*-butoxy, preferably methoxy.

In some embodiments, in $R^{X1}$, each halogen in the halogen and the $C_1$-$C_6$ alkyl substituted with one or more halogens is independently fluorine, chlorine, bromine, or iodine, preferably fluorine.

In some embodiments, in $R^{X1}$, the $C_1$-$C_6$ alkyl in the $C_1$-$C_6$ alkyl substituted with one or more halogens is methyl, ethyl, *n*-propyl, isopropyl, *n*-butyl, isobutyl, or *tert*-butyl, preferably methyl.

In some embodiments, in $R^5$, the $C_1$-$C_6$ alkoxy is methoxy, ethoxy, *n*-propoxy, isopropoxy, *n*-butoxy, isobutoxy, or *tert*-butoxy, preferably methoxy or ethoxy.

In some embodiments, in $R^{5-1}$ and $R^{5-2}$, each $C_1$-$C_6$ alkyl is independently methyl, ethyl, *n*-propyl, isopropyl, *n*-butyl, isobutyl, or *tert*-butyl, preferably methyl.

In some embodiments, in $R^6$, the halogen is fluorine, chlorine, bromine, or iodine, preferably fluorine.

In some embodiments, in $R^7$, the $C_1$-$C_6$ alkyl is methyl, ethyl, *n*-propyl, isopropyl, *n*-butyl, isobutyl, or *tert*-butyl, preferably methyl.

In some embodiments, in $R^8$, the $C_1$-$C_6$ alkyl is methyl, ethyl, *n*-propyl, isopropyl, *n*-butyl, isobutyl, or *tert*-butyl, preferably methyl.

In some embodiments, $R^1$ is $C_1$-$C_6$ alkyl, $C_3$-$C_6$ cycloalkyl, $C_1$-$C_6$ alkoxy, -$OR^{1-1}$, $C_1$-$C_6$ alkyl substituted with one or more $R^{1-2}$, or $C_1$-$C_6$ alkoxy substituted with one or more $R^{1-3}$.

In some embodiments, $R^2$ is halogen.

In some embodiments, $R^3$ is halogen.

In some embodiments, $R^4$ is hydrogen.

In some embodiments, $R^{5-1}$ is hydrogen.

In some embodiments, $R^{5-2}$ is hydrogen or $C_1$-$C_6$ alkyl.

In some embodiments, $R^6$ is halogen.

In some embodiments, $R^7$ is $C_1$-$C_6$ alkyl.

In some embodiments, $R^8$ is $C_1$-$C_6$ alkyl.

In some embodiments, in the compound of Formula I, $R^1$, $R^2$, and $R^3$ are each independently halogen, $C_1$-$C_6$ alkyl, $C_3$-$C_6$ cycloalkyl, $C_1$-$C_6$ alkoxy, -$OR^{1-1}$, $C_1$-$C_6$ alkyl substituted with one or more $R^{1-2}$, or $C_1$-$C_6$ alkoxy substituted with one or more $R^{1-3}$;

$R^{1-1}$ is $C_3$-$C_6$ cycloalkyl or 3- to 8-membered heterocycloalkyl; in the 3- to 8-membered heterocycloalkyl, the heteroatom is selected from 1, 2, or 3 types of N, O, and S, and the number of heteroatoms is 1, 2, or 3;
each $R^{1-2}$ is independently halogen;
each $R^{1-3}$ is independently $C_1$-$C_6$ alkoxy;
$X^1$ is $CR^{X1}$;
$R^{X1}$ is hydrogen, halogen, or $C_1$-$C_6$ alkyl substituted with one or more halogens;
$X^2$ is NH;
$R^4$ is hydrogen or $C_1$-$C_6$ alkyl;
$R^5$ is -OH, -$NR^{5-1}R^{5-2}$, or -CN;
$R^{5-1}$ and $R^{5-2}$ are each independently hydrogen or $C_1$-$C_6$ alkyl;
n is 0, 1, 2, or 3;
$R^6$ is hydrogen, halogen, hydroxyl, $C_1$-$C_6$ alkyl, $C_1$-$C_6$ alkyl-$C_1$-$C_6$ alkoxy, or $C_1$-$C_6$ alkoxy;
$R^7$ is hydrogen or $C_1$-$C_6$ alkyl;
$R^8$ is hydrogen, hydroxyl, $C_1$-$C_6$ alkyl, $C_1$-$C_6$ alkyl-$C_1$-$C_6$ alkoxy, or $C_1$-$C_6$ alkoxy.

In some embodiments, the compound of Formula I or the pharmaceutically acceptable salt thereof is a compound of Formula I-1 or a pharmaceutically acceptable salt thereof:

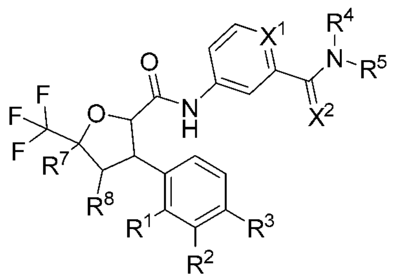

I-1 ;

$R^1$ is $C_1$-$C_6$ alkoxy or $C_1$-$C_6$ alkoxy substituted with one or more $R^{1-3}$;
each $R^{1-3}$ is independently $C_1$-$C_6$ alkoxy;
$R^2$ is halogen;
$R^3$ is halogen;
$X^1$ is N or $CR^{X1}$;
$R^{X1}$ is halogen;
$X^2$ is O or NH;
$R^4$ is hydrogen;
$R^5$ is -OH or -CN;
$R^7$ is $C_1$-$C_6$ alkyl;
$R^8$ is $C_1$-$C_6$ alkyl.

In some embodiments, $R^1$ is methyl, cyclopropyl, methoxy, -$CF_3$,

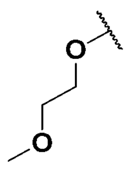 , 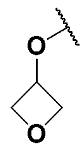 ,

or

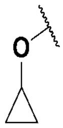.

In some embodiments, $R^2$ is fluorine.

In some embodiments, $R^3$ is fluorine.

In some embodiments, $X^1$ is N, $N^+$-$O^-$, CH, CF, or C-$CF_3$.

In some embodiments, $R^5$ is methoxy, ethoxy, -OH, -$NH_2$, -NH($CH_3$), or -CN.

In some embodiments,

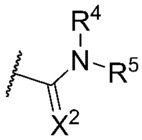

is

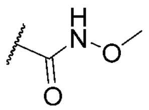, 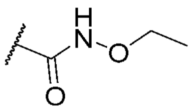, 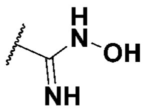,

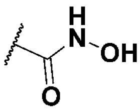, 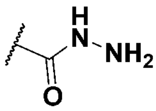, 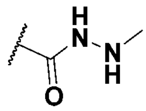, or 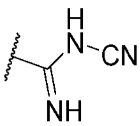.

In some embodiments, the compound of Formula I is any one of the following compounds:

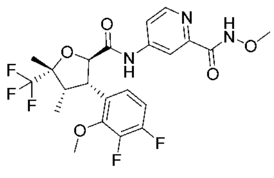

BX20-9-021

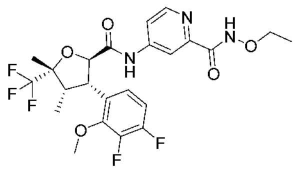

BX20-9-022

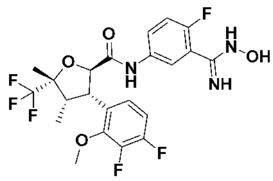

BX20-9-029

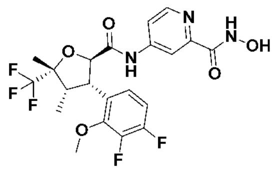

BX20-9-033

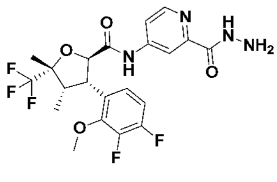

BX20-9-034

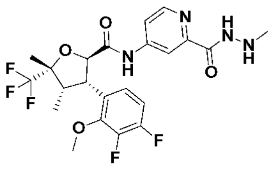

BX20-9-035

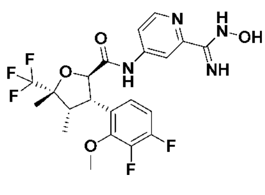

BX20-9-036

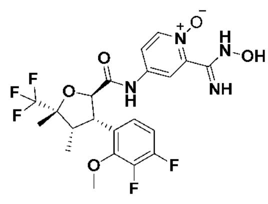

BX20-9-037

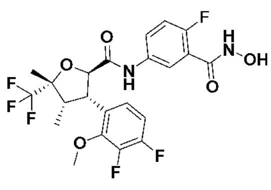

BX20-9-038

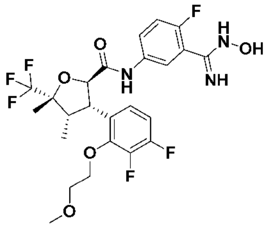

BX20-9-039

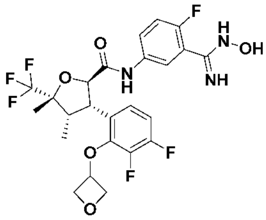

BX20-9-040

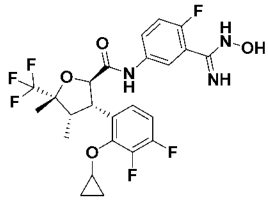

BX20-9-041

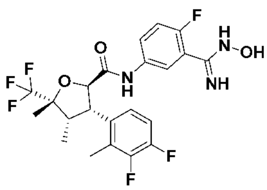

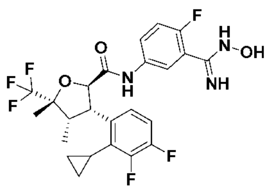

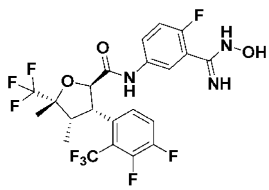

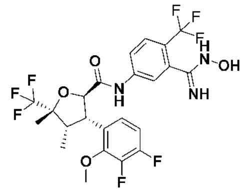

BX20-9-046

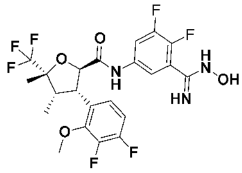

BX20-9-047

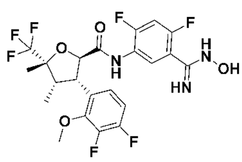

BX20-9-048

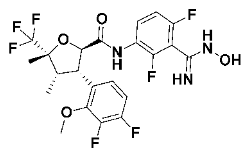

BX20-9-049

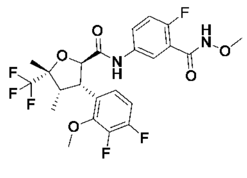

BX20-9-052

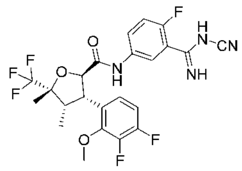

BX20-9-055

The present disclosure provides a pharmaceutical composition comprising:

(1) the compound of Formula I or the pharmaceutically acceptable salt thereof as described above, and
(2) a pharmaceutically acceptable excipient.

The present disclosure provides a use of substance A in the manufacture of a medicament for treating a disease; the disease may be pain, a pain-related disease, multiple sclerosis, incontinence, or arrhythmia;
the substance A is the compound of Formula I or the pharmaceutically acceptable salt thereof as described above, or the pharmaceutical composition as described above.
In some embodiments, the pain is one or more of acute pain, chronic pain, inflammatory pain, cancer pain, neuropathic pain, musculoskeletal pain, primary pain, intestinal pain, or idiopathic pain.
The present disclosure provides a use of substance A as described above in the manufacture of a medicament for inhibiting voltage-gated sodium channels; the voltage-gated sodium channel is preferably Nav1.8.
The present disclosure provides a use of substance A as described above in the manufacture of a voltage-gated sodium channel inhibitor; the voltage-gated sodium channel is preferably Nav1.8.
In some embodiments, the voltage-gated sodium channel inhibitor can be used in mammalian organisms; it can also be used *in vitro*, mainly for experimental purposes, for example: provided as a standard sample or control sample for comparison, or prepared into a kit according to conventional methods in the art to provide rapid detection of the effect of inhibiting voltage-gated sodium channels.
The present disclosure provides a use of substance A as described above in the manufacture of a medicament for a disease caused by abnormal activation of voltage-gated sodium channels; the voltage-gated sodium channel is preferably Nav1.8; the disease may be pain, a pain-related disease, multiple sclerosis, incontinence, or arrhythmia.
The present disclosure further provides a preparation method for a compound of Formula I: it is obtained through the following Route I or Route II:

Route I:

condensing the general formulas **(Z1)** and **(Z2)** to obtain the general formula **(Z3)**, reducing the general formula **(Z3)** to obtain the general formula **(Z4)**, reducing the general formula **(Z4)** by DIBAL-H to obtain the general formula **(Z5)**, reacting the general formula **(Z5)** with acetyl chloride to obtain the general formula **(Z6)**, reacting the general formula **(Z6)** under TMSCN/$BF_3OEt_2$ conditions to obtain the general formula **(Z7)**, hydrolyzing the general formula **(Z7)** to obtain the general formula **(Z8)**, condensing the general formula **(Z8)** with amine to obtain the general formula **(Z9)**, reacting the general formula **(Z9)** under conditions such as ammonia-methanol or hydroxylamine to obtain the compound of Formula I:

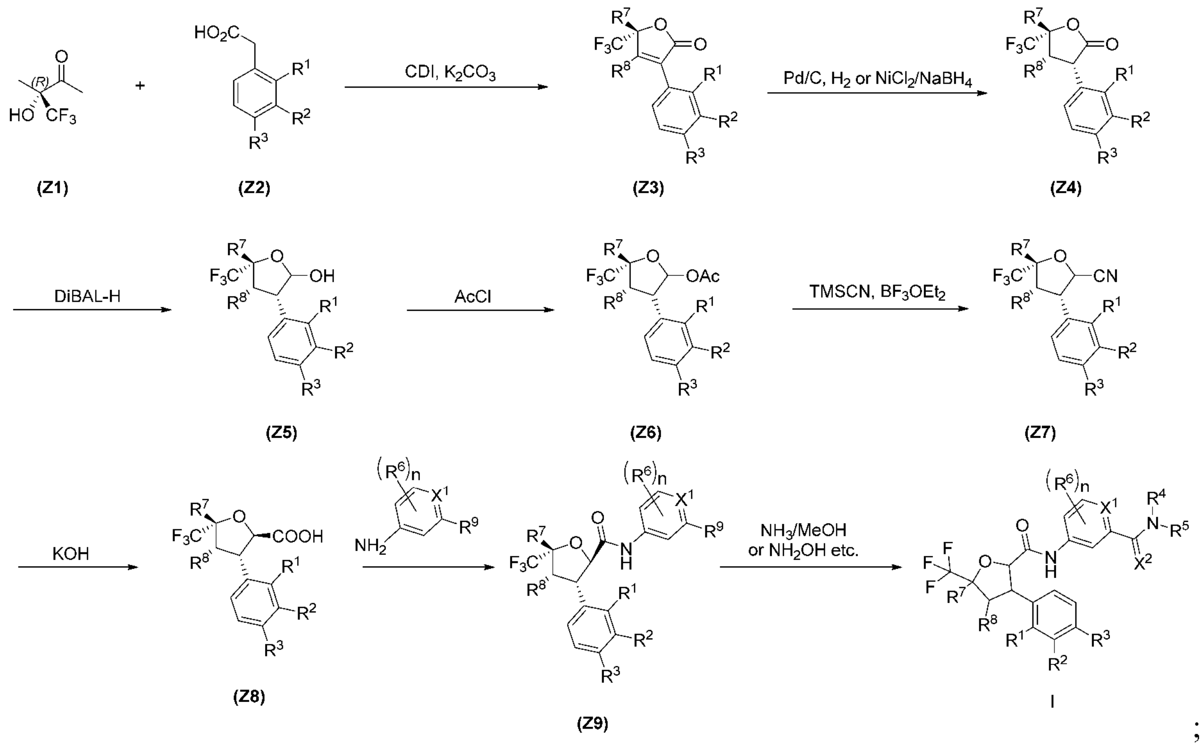


;

Route II:

reacting the general formula **(Z7)** under boron tribromide conditions to obtain the general formula **(Z10)**, performing a nucleophilic substitution reaction of the general formula **(D10)** with alkyl halides or alkyl sulfonates to obtain the general formula **(Z11)**, hydrolyzing the general formula **(Z11)** to obtain the general formula **(Z12)**, condensing the general formula **(Z12)** with amine to obtain the general formula **(Z13)**, reacting the general formula **(Z13)** under conditions such as ammonia-methanol or hydroxylamine to obtain the compound of Formula I:

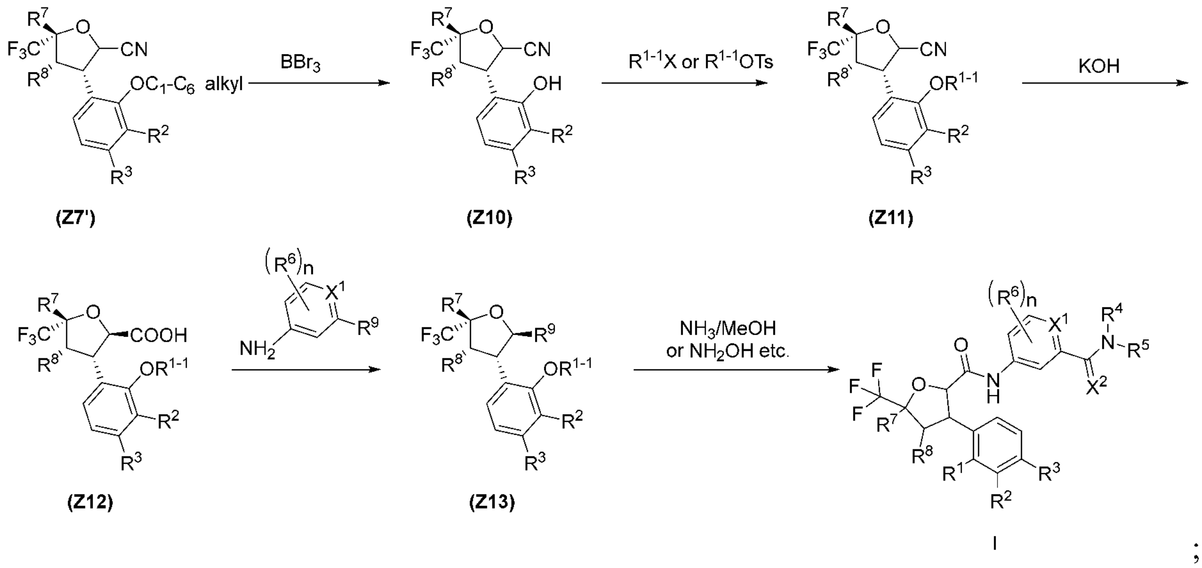


(Z7') (Z10) (Z11)

(Z12) (Z13) I

;

wherein $R^9$ is $-C(=O)OC_1-C_6$ alkyl or CN;

$R^2$, $R^3$, $X^1$, $X^2$, $R^4$, $R^5$, $R^6$, $R^7$, $R^8$, and n are as defined in any one of the above;

when $R^1$ is halogen, $C_1-C_6$ alkyl, $C_3-C_6$ cycloalkyl, $C_1-C_6$ alkoxy, or $C_1-C_6$ alkyl substituted with one or more $R^{1-2}$, the compound of Formula I is prepared via Route I;

when $R^1$ is $OR^{1-1}$ or $C_1-C_6$ alkoxy substituted with one or more $R^{1-3}$, the compound of Formula I is prepared via Route II.

## Terminology explanation

In the present disclosure, the term "pharmaceutically acceptable salt" refers to a salt obtained by the reaction of the compound with a pharmaceutically acceptable acid or base. When the compound contains a relatively acidic functional group, a base addition salt can be obtained by contacting the compound with a sufficient amount of a pharmaceutically acceptable base in a suitable inert solvent. When the compound contains a relatively basic functional group, an acid addition salt can be obtained by contacting the compound with a sufficient amount of a pharmaceutically acceptable acid in a suitable inert solvent. For details, refer to Handbook of Pharmaceutical Salts: Properties, Selection, and Use (P. Heinrich Stahl, Camille G. Wermuth, 2011, 2nd Revised Edition).

In the present disclosure,

“ 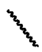 ”

in the structural moiety refers to that the structural moiety is connected to the rest of the molecule through this bond. For example,

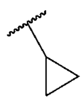

refers to cyclopropyl.

In the present disclosure, the "-" at the end of a group indicates that the group is connected to the rest of the molecule through this site. For example, -OH refers to a hydroxyl group.

In the present disclosure, the term "one or more" refers to 1, 2, 3, 4, or 5, for example 1, 2, or 3.

In the present disclosure, the term "halogen" refers to fluorine, chlorine, bromine, or iodine.

In the present disclosure, the term "alkyl" refers to a straight-chain or branched, saturated monovalent hydrocarbon group with a specified number of carbon atoms (*e.g.*, $C_1-C_6$). The alkyl includes but is not limited to: methyl, ethyl, *n*-propyl, isopropyl, *n*-butyl, isobutyl, *sec*-butyl, *tert*-butyl, *n*-pentyl, *n*-hexyl, and the like.

In the present disclosure, the term "alkoxy" refers to the group $R^Y$-O-, wherein the definition of $R^Y$ is the same as that of the term "alkyl". The alkoxy includes but is not limited to: methoxy, ethoxy, *n*-propoxy, isopropoxy, and the like.

In the present disclosure, the term "cycloalkyl" refers to a cyclic, saturated monovalent hydrocarbon group having

a specified number of carbon atoms (*e.g.*, $C_3$-$C_6$). The cycloalkyl includes but is not limited to:

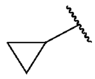, 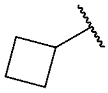, 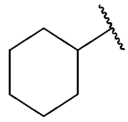,

and the like.

In the present disclosure, the term "heterocycloalkyl" refers to a cyclic, saturated monovalent group having a specified number of ring atoms (*e.g.*, 3- to 12-membered, 4- to 8-membered, 5-membered, 6-membered, or 7-membered), a specified number of heteroatoms (*e.g.*, 1, 2, or 3), and specified types of heteroatoms (one or more of N, O, and S). The heterocycloalkyl is connected to the remainder of the molecule through a carbon atom or a heteroatom. The heterocycloalkyl includes but is not limited to:

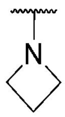, 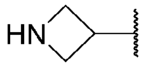,

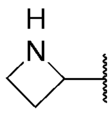, 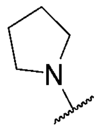, 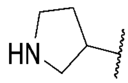, 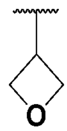,

and the like.

In the present disclosure, the term "pharmaceutically acceptable excipient" refers to all substances contained in a pharmaceutical preparation other than the active pharmaceutical ingredient, which are generally divided into two major categories: vehicles and additives. For details, refer to Pharmacopoeia of the People's Republic of China (2020 Edition) and Handbook of Pharmaceutical Excipients (Paul J Sheskey, Bruno C Hancock, Gary P Moss, David J Goldfarb, 2020, 9th Edition).

On the basis of not violating the common knowledge in the art, the above-mentioned preferred conditions may be arbitrarily combined to obtain various preferred embodiments of the present disclosure.

The reagents and raw materials used in the present disclosure are all commercially available.

The positive progressive effects of the present disclosure lie in that the compounds of the present disclosure have one or more of the following effect advantages: (1) novel structure; (2) good blocking effect (inhibition) on Nav1.8 channel activity; (3) good selectivity toward other Nav subtypes (*e.g.*, Nav1.1, Nav1.2, Nav1.3, Nav1.4, Nav1.5, Nav1.6, Nav1.7, Nav1.9) with high safety.

## DETAILED DESCRIPTION OF THE PREFERRED EMBODIMENT

The present disclosure will be further illustrated below by way of examples, but the present disclosure is not limited to the scope of the examples described herein. Experimental methods in the following examples, for which no specific conditions are indicated, are performed according to conventional methods and conditions, or are selected in accordance with product manuals.

### **Example 1:** Synthesis of **BX20-9-021**

Step 1: Synthesis of intermediate **A2**

The synthesis route is shown in the following formula:

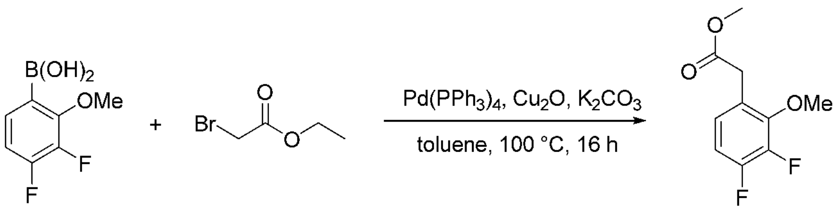


A1 SM1 A2

Method: Compound **A1** (18.00 g, 95.76 mmol) was dissolved in toluene (300 mL), followed by sequential addition of **SM1** (13.32 g, 87.07 mmol), $Pd(PPh_3)_4$ (3.19 g, 2.76 mmol), $Cu_2O$ (0.37 g, 2.62 mmol), and $K_2CO_3$ (41.19 g, 298.06 mmol). The reaction mixture was purged with $\mathbf{N_2}$ and stirred at 100°C for 16 hours. After the reaction was complete, the reaction mixture was filtered through diatomite and washed with EA. The filtrate was collected, extracted with saturated brine, and the organic phase was dried. After concentration under reduced pressure, the residue was purified by column chromatography to obtain compound **A2** (7.60 g, 40.4%) as a white solid.

Step 2: Synthesis of intermediate **A3**

The synthesis route is shown in the following formula:

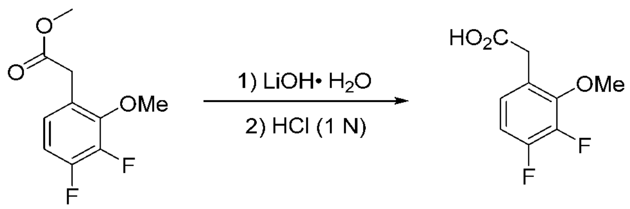


A2 A3

Method: Compound **A2** (7.60 g, 35.16 mmol) was dissolved in THF (80 mL), followed by the addition of an aqueous lithium hydroxide solution (3.39 g $LiOH{\cdot}H_2O$ dissolved in 40 mL $H_2O$, 80.87 mmol). The reaction mixture was stirred at 50°C for 3 hours. After the reaction was complete, the mixture was diluted with DCM and partitioned. The mixture was extracted three times with $H_2O$, and the aqueous phases were combined. The pH was adjusted to pH < 5 with 1 N HCl, followed by three extractions with DCM. The organic phases were combined, dried, and concentrated under reduced pressure to obtain compound **A3** (5.90 g, 83.0%) as a white solid.

Step 3: Synthesis of intermediate **S2**

The synthesis route is shown in the following formula:

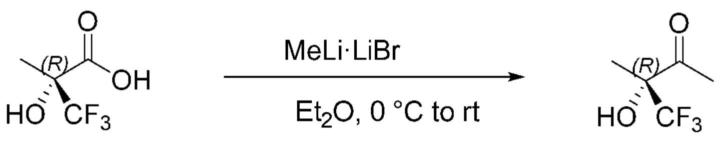


**S1** **S2**

Method: Compound **S1** (15.50 g, 98.05 mmol) was dissolved in $Et_2O$ (155 mL), and the system was purged with $N_2$. The temperature was lowered to around 0°C, and MeLi·LiBr (209 mL, 313.76 mmol, 1.5 M in $Et_2O$) was added in batches while maintaining the temperature between 0-10°C. After the addition was complete, the reaction mixture was allowed to warm to room temperature and stirred for 12 hours. After the reaction was complete, the reaction mixture was quenched by dropwise addition of $H_2O$ (100 mL) and saturated sodium chloride solution (20 mL), followed by stirring with citric acid (25.04 g) for 0.5 hours. The mixture was extracted three times with $Et_2O$. The organic phases were combined, dried, and concentrated under reduced pressure to 30 g, yielding crude **S2** (about 35% in $Et_2O$, 30 g).

Step 4: Synthesis of intermediate **S3**

The synthesis route is shown in the following formula:

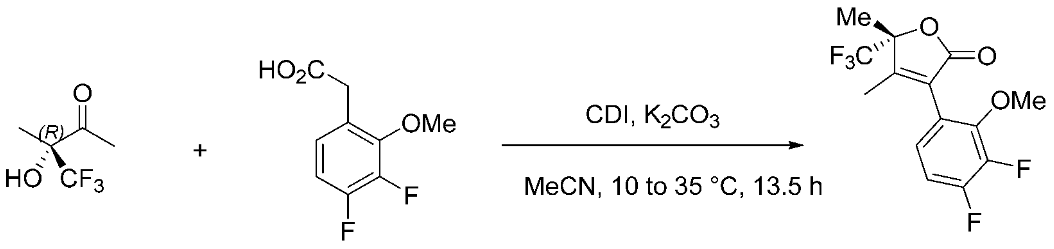


S2 A3 S3

Method: Compound **A3** (5.90 g, 29.20 mmol) was dissolved in MeCN (210 mL) and stirred until clear. The solution was cooled in an ice-water bath, and CDI (7.00 g, 30.70 mmol) was added. The system was purged with $N_2$, and the mixture was stirred at 10°C or lower for 1.5 hours. **S2** (about 35% in $Et_2O$, 15 g, 30 mmol) and $K_2CO_3$ (5.00 g, 36.50 mmol) were added. The reaction mixture was stirred at 35°C for 12 hours. After the reaction was complete, water and dilute hydrochloric acid were added to the reaction mixture and stirred until clear. The mixture was extracted with EA, washed with concentrated brine, dried over anhydrous sodium sulfate, and concentrated. The residue was purified by column chromatography to obtain **S3** (7.40 g, 78.7%) as a white solid. MS (ESI, m/z) 323 $[M+H]^+$.

Step 5: Synthesis of **S4**

The synthesis route is shown in the following formula:

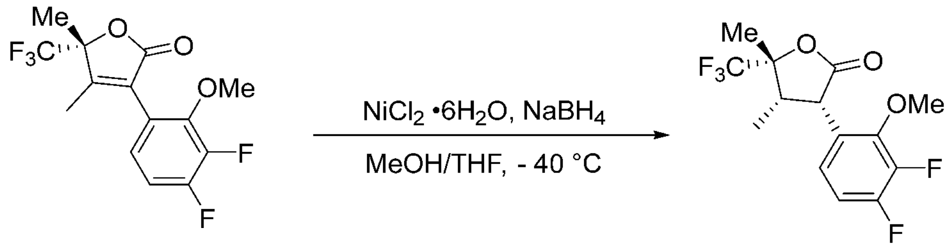


S3 S4

Method: Compound **S3** (9.00 g, 28.0 mmol) was dissolved in anhydrous methanol (800 mL) and anhydrous tetrahydrofuran (160 mL) and stirred until clear. The solution was cooled to -40°C, and the first batch of $NiCl_2 \cdot 6H_2O$ (6.80 g, 28.6 mmol) and $NaBH_4$ (5.60 g, 148.4 mmol) was added, followed by the second batch of $NiCl_2 \cdot 6H_2O$ (6.80 g, 28.6 mmol) and $NaBH_4$ (5.60 g, 148.4 mmol). The reaction completed immediately after addition. After the reaction was complete, saturated ammonium chloride solution was slowly added dropwise to the reaction mixture at -40°C and stirred for 30 minutes. The mixture was allowed to warm to room temperature, filtered through diatomite, extracted with DCM, washed with concentrated brine, dried over anhydrous sodium sulfate, and concentrated. The residue was purified by column chromatography to obtain compound **S4** (3.57 g, 39.3%) as a colorless oil. MS (ESI, m/z) 325 $[M+H]^+$.

The NMR spectrum is:

$^1H$ NMR (400 MHz, $CDCl_3$) $\delta$ = 6.96-6.83 (m, 2H), 4.47 (d, $J$ = 9.6 Hz, 1H), 4.02 (d, $J$ = 2.8 Hz, 1H), 2.92-2.84 (m, 1H), 1.70 (s, 3H), 0.81-0.78 (m, 3H).

Step 6: Synthesis of **S5**

The synthesis route is shown in the following formula:

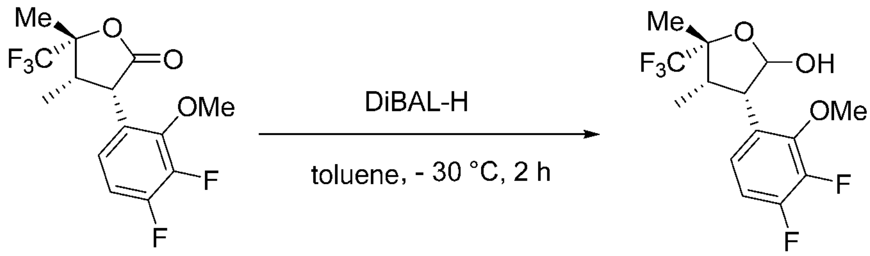


S4 S5

Method: Compound **S4** (3.57 g, 11.0 mmol) was dissolved in toluene (30 mL), and the system was purged with $N_2$. The solution was cooled to -30°C, and DiBAL-H (1.5 M, 7.7 mL, 11.6 mmol) was added dropwise slowly. The reaction mixture was stirred at -25 to -30°C for 2 hours. After the reaction was complete, the reaction mixture was quenched by adding to saturated ammonium chloride solution, filtered through diatomite, extracted with EA, washed with concentrated brine, dried over anhydrous sodium sulfate, and concentrated under reduced pressure to obtain compound **S5** (3.53 g, crude) as a colorless oil. MS (ESI, m/z) 327 $[M+H]^+$.

Step 7: Synthesis of **S6**

The synthesis route is shown in the following formula:

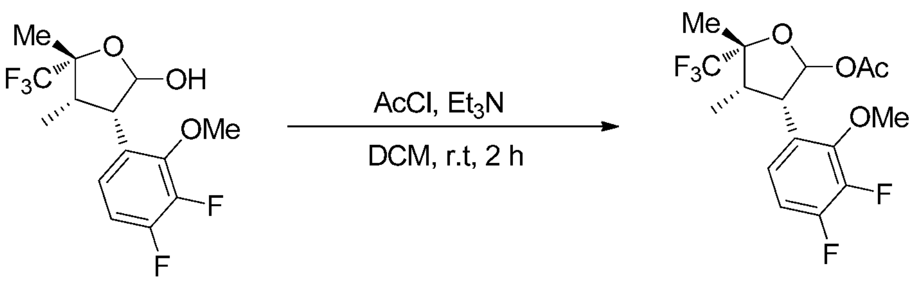


S5 S6

Method: Compound **S5** (3.53 g, 10.8 mmol) was dissolved in anhydrous DCM (30 mL) and stirred until clear. $Et_3N$ (2.18 g, 21.6 mmol) was added, and the solution was cooled to 0-10°C in an ice-water bath. AcCl (1.70 g, 21.6 mmol) was added dropwise slowly. After the dropwise addition was complete, the reaction mixture was warmed to room temperature and stirred for 1 hour. After the reaction was complete, the reaction mixture was quenched with saturated ammonium chloride solution, extracted with DCM, washed with concentrated brine, dried over anhydrous sodium sulfate, and concentrated to obtain compound **S6** (4.36 g, crude) as a colorless oil. MS (ESI, m/z) 369 $[M+H]^+$.

Step 8: Synthesis of **S7**

The synthesis route is shown in the following formula:

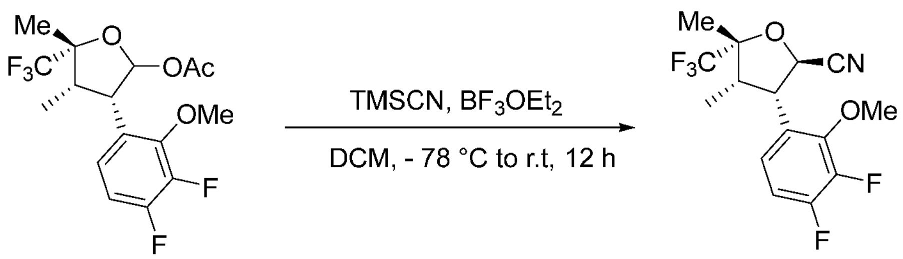


S6 S7

Method: Compound **S6** (4.36 g, 11.8 mmol) was dissolved in anhydrous DCM (40 mL) and stirred until clear. The system was purged with $N_2$ and cooled to -78°C. TMSCN (3.52 g, 35.5 mmol) and $BF_3OEt_2$ (5.04 g, 35.5 mmol) were added

dropwise sequentially. After the dropwise addition was complete, the reaction mixture was stirred at -60°C for 1 hour and then allowed to warm to room temperature and stirred for 12 hours. After the reaction was completed, the reaction mixture was quenched with saturated sodium carbonate solution, filtered through diatomite, extracted with DCM, washed with concentrated brine, dried over anhydrous sodium sulfate, concentrated, and purified by column chromatography to obtain compound **S7** (2.25 g, 56.9%) as a colorless oil. MS (ESI, m/z) 336 $[M+H]^+$.

Step 9: Synthesis of **S8**

The synthesis route is shown in the following formula:

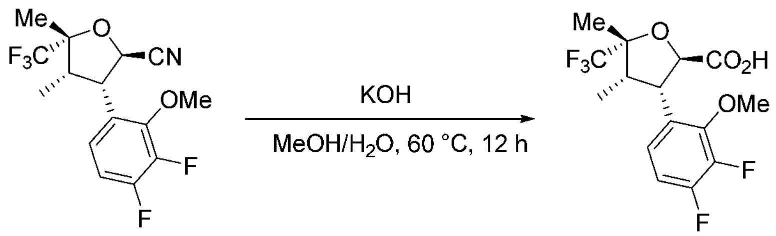


**S7** **S8**

Method: Compound **S7** (2.25 g, 6.7 mmol) was dissolved in MeOH (50 mL), and an aqueous KOH solution (2.63 g KOH dissolved in 10 mL $H_2O$, 46.9 mmol) was added. The reaction mixture was stirred at 60°C for 12 hours. After the reaction was complete, the mixture was extracted with MTBE. The aqueous phase was adjusted to pH < 5 with hydrochloric acid and extracted with MTBE. The organic phase was washed with concentrated brine, dried over anhydrous sodium sulfate, and concentrated to obtain compound **S8** (2.20 g, crude) as a colorless oil. MS (ESI, m/z) 355 $[M+H]^+$.

Step 10: Synthesis of intermediate **S10**

The synthesis route is shown in the following formula:

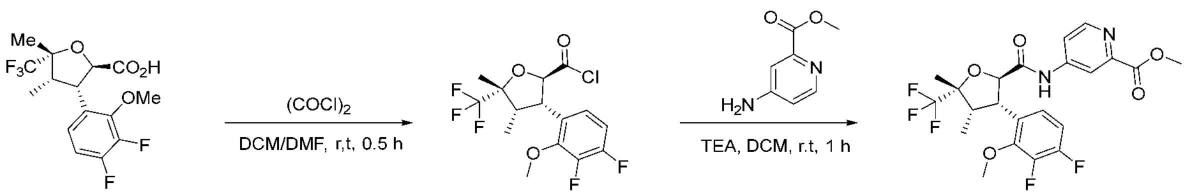


**S8** **S9** **S10**

Method: **S8** (150 mg, 0.42 mmol) was dissolved in DCM (5 mL) and DMF (20 μL). Oxalyl chloride (0.3 mL, 3.20 mmol) was added dropwise slowly, and the mixture was stirred at room temperature for 0.5 hours. The reaction mixture was concentrated under reduced pressure at 40°C, then redissolved in DCM (5 mL) and slowly added dropwise to a solution of methyl 4-aminopyridine-2-carboxylate (140 mg, 0.91 mmol) and TEA (142 mg, 1.4 mmol) in DCM (5 mL). The reaction mixture was stirred at room temperature for 1 hour. After the reaction was complete, the reaction mixture was quenched with saturated $NH_4Cl$ solution (30 mL) and extracted with DCM (30 mL × 3). The organic phases were combined, washed with saturated NaCl solution, dried over anhydrous $Na_2SO_4$, and concentrated to obtain compound **S9** (390 mg, crude) as a yellow oil. MS (ESI, m/z) 489 $[M+H]^+$.

Step 11: Synthesis of intermediate **S11**

The synthesis route is shown in the following formula:

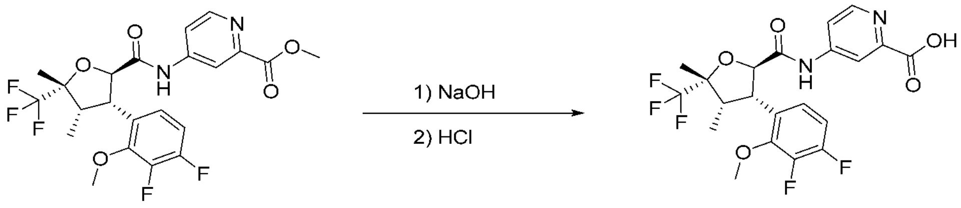


S10 S11

Method: **S10** (390 mg, 0.80 mmol) was dissolved in methanol (15 mL), and NaOH (1 N, 15 mL) was added. The mixture was stirred at room temperature overnight. After the reaction was complete, the pH was adjusted to 3-4. The mixture was extracted with EA, dried over sodium sulfate, and concentrated to obtain **S11** (300 mg, crude). MS (ESI, m/z) 475 $[M+H]^+$.

Step 12: Synthesis of **BX20-9-021**

The synthesis route is shown in the following formula:

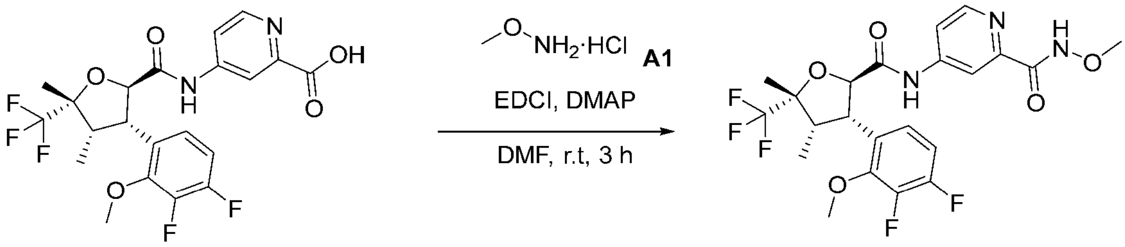


S11 BX20-9-021

Method: **S11** (150 mg, 0.31 mmol) was dissolved in DMF (3 mL), followed by the addition of EDCI (96 mg, 0.5 mmol), DMAP (122 mg, 1 mmol), and **A1** (42 mg, 0.5 mmol). The reaction mixture was stirred at room temperature for 3 hours. After the reaction was complete, the reaction mixture was quenched with water, extracted with EA, dried over sodium sulfate, concentrated, purified by preparative chromatography, and lyophilized to obtain **BX20-9-021** (13 mg, yield: 8.4%) as a light yellow solid. MS (ESI, m/z) 504 $[M+H]^+$.

$^1$H NMR (400 MHz, $CD_3OD$) $\delta$ = 8.45 (d, $J$ = 5.6 Hz, 1H), 8.22 (d, $J$ = 2.4 Hz, 1H), 7.87 (dd, $J$ = 5.6 Hz, 2.4 Hz, 1H), 7.13-7.09 (m, 1H), 7.00-6.94 (m, 1H), 5.07 (d, $J$ = 10.4 Hz, 1H), 4.32 (dd, $J$ = 10.4 Hz, 8.0 Hz, 1H), 3.99 (d, $J$ = 2.4 Hz, 3H), 3.80 (s, 3H), 2.83-2.75 (m, 1H), 1.65 (s, 3H), 0.82-0.79 (m, 3H).

**Example 2: Synthesis of BX20-9-022**

Step 1: Synthesis of intermediate S11

Steps 1 to 11 of Example 1 were referenced

Step 2: Synthesis of **BX20-9-022**

The synthesis route is shown in the following formula:

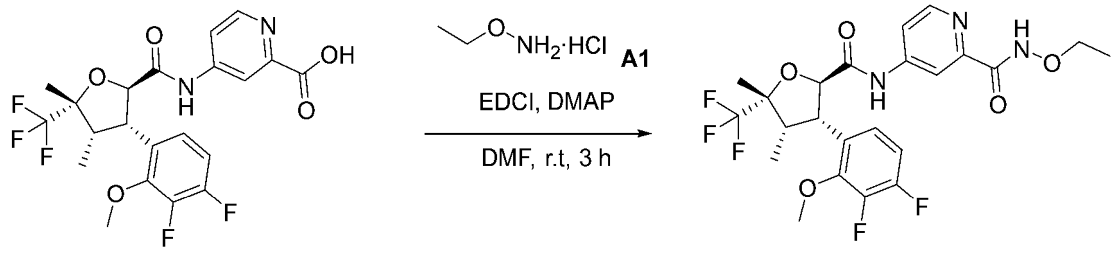


**S11** **BX20-9-022**

Method: **S11** (150 mg, 0.31 mmol) was dissolved in DMF (3 mL), followed by the addition of EDCI (96 mg, 0.5 mmol), DMAP (122 mg, 1 mmol), and **A1** (49 mg, 0.5 mmol). The reaction mixture was stirred at room temperature for 3 hours. After the reaction was complete, the reaction mixture was quenched with water, extracted with EA, dried over sodium sulfate, concentrated, purified by preparative chromatography, and lyophilized to obtain **BX20-9-021** (13 mg, yield: 8.1%) as a light yellow solid. MS (ESI, m/z) 518 $[M+H]^+$.

$^1H$ NMR (400 MHz, $CD_3OD$) $\delta$ = 8.45 (d, *J* = 5.6 Hz, 1H), 8.22 (d, *J* = 2.4 Hz, 1H), 7.87 (dd, *J* = 5.6 Hz, 2.4 Hz, 1H), 7.13-7.09 (m, 1H), 7.00-6.94 (m, 1H), 5.07 (d, *J* = 10.4 Hz, 1H), 4.32 (dd, *J* = 10.4 Hz, 8.0 Hz, 1H), 4.04-3.98 (m, 5H), 3.80 (s, 3H), 2.83-2.74 (m, 1H), 1.65 (s, 3H), 1.29 (t, *J* = 6.8 Hz, 3H), 0.82-0.79 (m, 3H).

**Example 3: Synthesis of BX20-9-029**

Step 1: Synthesis of intermediate S8

Steps 1-9 of Example 1 were referenced

Step 2: Synthesis of intermediate S9'

The synthesis route is shown in the following formula:

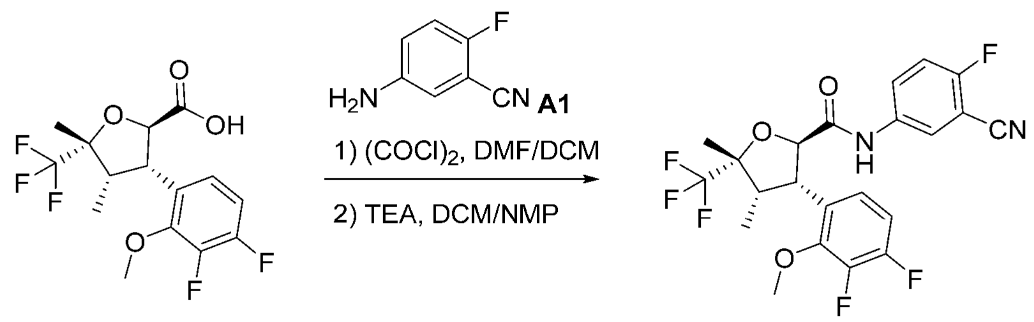


**S8** **S9'**

Method: **S8** (150 mg, 0.424 mmol) was dissolved in DCM (6 mL), followed by the addition of 1 drop of DMF. The mixture was cooled to 0°C, and oxalyl chloride (0.1 mL, 1.272 mmol) was added dropwise slowly. The reaction mixture was stirred at room temperature for 0.5 hours. After the reaction was complete, the reaction mixture was concentrated under reduced pressure, redissolved in DCM (5 mL), and slowly added dropwise to a solution of **A1** (69 mg, 0.509 mmol) and TEA (154 mg, 1.527 mmol) in DCM/NMP (10:1, 5.5 mL). The reaction mixture was stirred at room temperature for 0.5 hours. After the reaction was complete, the reaction mixture was concentrated under reduced pressure, redissolved in EA, and quenched by dropwise addition of water (15 mL). The mixture was extracted with EA, and the organic phases were combined, washed once with water and once with saturated NaCl solution, dried over anhydrous $Na_2SO_4$, and concentrated under reduced pressure to obtain compound **S9'** (250 mg, crude) as a yellow oil. MS (ESI, m/z) 473 $[M+H]^+$.

Step 3: Synthesis of **BX20-9-029**

The synthesis route is shown in the following formula:

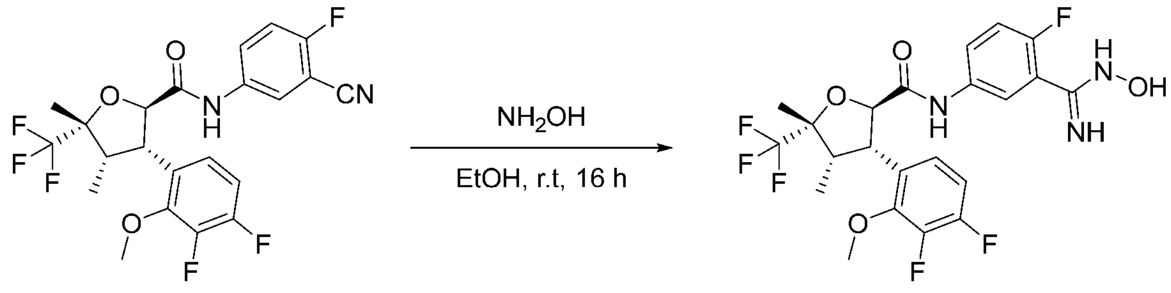


S9' BX20-9-029

Method: Compound **S9'** (250 mg, crude, 0.424 mmol) was dissolved in EtOH (6 mL), and $NH_2OH$ (112 mg, 1.696 mmol, 50%) was added. The reaction mixture was stirred at 80°C for 1 hour. After the reaction was complete, the reaction mixture was concentrated under reduced pressure and purified by preparative chromatography (formic acid system), followed by lyophilization to obtain **BX20-9-029** (128 mg, 60%) as a white solid. MS (ESI, m/z) 497 $[M+H]^+$.

$^1H$ NMR (400 MHz, DMSO-$d_6$) $\delta$ 10.33 (s, 1H), 9.62 (s, 1H), 7.75 (dd, *J* = 6.8 Hz, 2.8 Hz, 1H), 7.67-7.62 (m, 1H), 7.21-7.12 (m, 3H), 5.77 (s, 2H), 5.04 (d, *J* = 10.4 Hz, 3H), 4.27-4.20 (m, 1H), 3.94 (d, *J* = 2.0 Hz, 3H), 2.79-2.71 (m, 1H), 1.59 (s, 3H), 0.75-0.69 (m, 3H).

**Example 4: Synthesis of BX20-9-033**

Step 1: Synthesis of intermediate **S10**

The synthesis was carried out with reference to Steps 1 to 10 of Example 1

Step 2: Synthesis of compound **BX20-9-033**

The synthesis route is shown in the following formula:

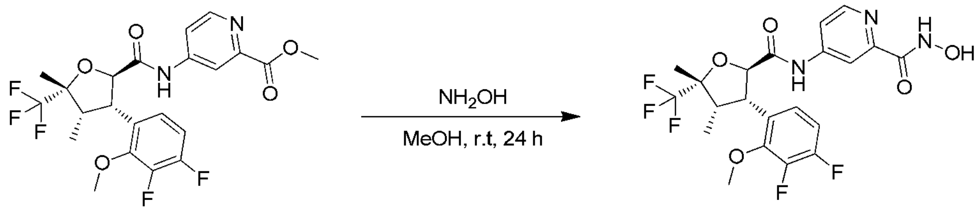


S10 BX20-9-033

Method: Compound **S10** (180 mg, 0.37 mmol) was dissolved in MeOH (8 mL), and $NH_2OH$ (50% in water, 220 mg, 3.4 mmol) was added dropwise. The reaction mixture was stirred at room temperature for 24 hours. After the reaction was complete, the reaction mixture was concentrated under reduced pressure at 40°C and purified by preparative chromatography (formic acid system), followed by lyophilization to obtain **BX20-9-033** (58 mg, yield: 32.2%) as a white solid powder. MS (ESI, m/z) 489 $[M+H]^+$.

$^1H$ NMR (400MHz, DMSO-$d_6$) $\delta$ = 11.39 (s,1H), 10.74 (s, 1H), 9.09 (s, 1H), 8.45 (d, *J* = 5.2 Hz, 1H), 8.23 (d, *J* = 2.0 Hz, 1H), 7.81 (dd, *J* = 5.2 Hz, *J* = 2.0 Hz, 1H), 7.21-7.13 (m, 2H), 5.11 (d, *J* = 10.4 Hz, 1H), 4.22 (dd, *J* = 10.4 Hz, *J* = 8.0 Hz, 1H), 3.95 (d, *J* = 2.0 Hz, 3H), 2.81-2.73 (m, 1H), 1.61 (s, 3H), 0.74-0.72 (m, 3H).

**Example 5: Synthesis of BX20-9-034**

Step 1: Synthesis of intermediate **S10**

The synthesis was carried out with reference to Steps 1 to 10 of Example 1

Step 2: Synthesis of compound **BX20-9-034**

The synthesis route is shown in the following formula:

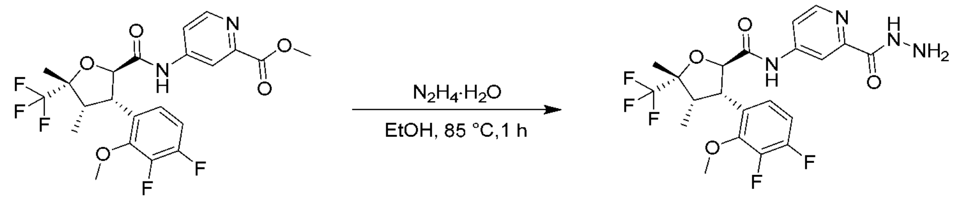


S10

BX20-9-034

Method: Compound **S10** (180 mg, 0.37 mmol) was dissolved in EtOH (2 mL), and $N_2H_4 \cdot H_2O$ (50 mg, 1.0 mmol) was added dropwise. The reaction mixture was stirred at 85°C for 1 hour. After the reaction was complete, the reaction mixture was concentrated under reduced pressure and purified by preparative chromatography, followed by lyophilization to obtain **BX20-9-034** (62 mg, yield: 34.4%) as a white solid powder. MS (ESI, m/z) 488 $[M+H]^+$.

$^1H$ NMR (400MHz, DMSO-$d_6$) $\delta$ = 10.78 (s,1H), 9.84 (s, 1H), 8.47 (d, $J$ = 5.6 Hz, 1H), 8.25 (s, 1H), 7.82 (dd, $J$ = 5.6 Hz, $J$ = 2.0 Hz, 1H), 7.20-7.16 (m, 2H), 5.11 (d, $J$ = 10.0 Hz, 1H), 4.28-4.23 (m, 1H), 3.95 (d, $J$ = 2.0 Hz, 3H), 2.79-2.76 (m, 1H), 1.61 (s, 3H), 0.74-0.72 (m, 3H).

**Example 6: Synthesis of BX20-9-035**

Step 1: Synthesis of intermediate **S11**

The synthesis was carried out with reference to Steps 1 to 11 of Example 1

Step 2: Synthesis of intermediate **35-3**

The synthesis route is shown in the following formula:

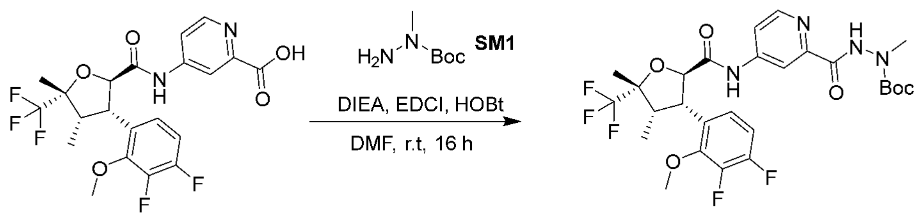


S11

35-3

Method: Compound **S11** (175 mg, 0.36 mmol) was dissolved in DMF (5 mL), followed by sequential addition of DIEA (160 mg, 1.24 mmol), EDCI (138 mg, 0.72 mmol), and HOBt (60 mg, 0.44 mmol). After stirring at room temperature for 10 min, **SM1** (53 mg, 0.3 mmol) was then added, and the reaction mixture was stirred at room temperature for 16 hours. After the reaction was complete, the reaction mixture was extracted with EA (30 mL × 3). The organic phases were combined, washed with saturated brine, dried over anhydrous $Na_2SO_4$, and concentrated under reduced pressure to obtain **35-3** (210 mg, crude) as a yellow oily liquid. MS (ESI, m/z) 602 $[M+H]^+$.

Step 3: Synthesis of compound **BX20-9-035**

The synthesis route is shown in the following formula:

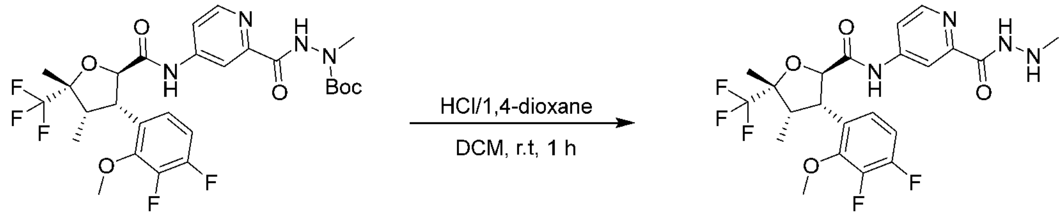


35-3 BX20-9-035

Method: Compound **35-3** (210 mg, 0.35 mmol) was dissolved in DCM (2 mL), and HCl/1,4-dioxane (1.75 mL, 6.98 mmol) was added dropwise. The reaction mixture was stirred at room temperature for 1 hour. After the reaction was complete, the reaction mixture was concentrated under reduced pressure and purified by preparative chromatography, followed by lyophilization to obtain **BX20-9-035** (23 mg, yield: 13.1%) as a white solid powder. MS (ESI, m/z) 502.2 $[M+H]^+$.

$^1$H NMR (400MHz, DMSO-$d_6$) $\delta$ = 10.76 (s,1H), 10.13 (s, 1H), 8.47 (d, $J$ = 5.6 Hz, 1H), 8.24 (d, $J$ = 2.0 Hz, 1H), 7.83 (dd, $J$ = 5.6 Hz, $J$ = 2.0 Hz, 1H), 7.21-7.13 (m, 2H), 5.11 (d, $J$ = 10.4 Hz, 1H), 4.25 (dd, $J$ = 10.4 Hz, $J$ = 8.0 Hz, 1H), 3.95 (d, $J$ = 2.0 Hz, 3H), 2.81-2.73 (m, 1H), 2.52 (s, 3H), 1.61 (s, 3H), 0.74-0.72 (m, 3H).

**Example 7: Synthesis of BX20-9-036**

Step 1: Synthesis of **S8**

The synthesis was carried out with reference to Steps 1 to 9 of Example 1

Step 2: Synthesis of **S9**

The synthesis route is shown in the following formula:

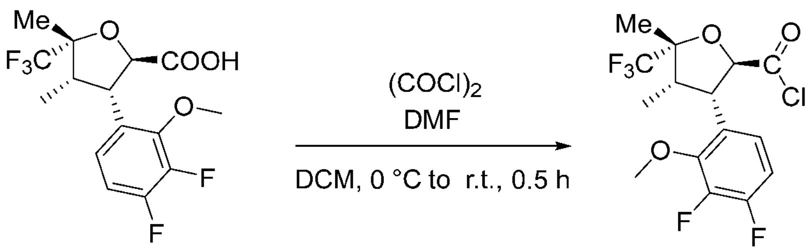


**S8** **S9**

Method: Compound **S8** (0.15 g, 0.42 mmol) was dissolved in DCM (4 mL) and stirred until clear. DMF (0.1 mL) was added, and the mixture was cooled to 0°C in an ice-water bath. Oxalyl chloride (0.11 g, 0.84 mmol) was added dropwise. After the addition was complete, the mixture was warmed to room temperature and stirred for 0.5 hours. The reaction mixture was concentrated under reduced pressure to obtain crude **S9**, which was used directly in the next step.

Step 3: Synthesis of **36-1**

The synthesis route is shown in the following formula:

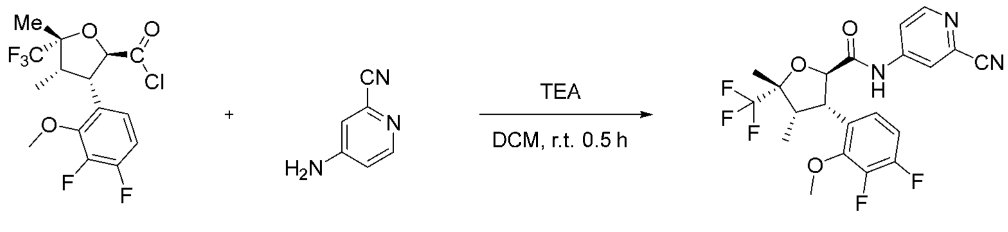


**S9** **SM1** **36-1**

Method: Compound **SM1** (76 mg, 0.64 mmol) was dissolved in NMP (2 mL), and TEA (86 mg, 0.85 mmol) was added. **S9** (crude, diluted with 5 mL DCM, 0.42 mmol) was then added slowly. The reaction mixture was stirred at room temperature for 0.5 hours. After the reaction was complete, the reaction mixture was quenched with saturated ammonium chloride solution and extracted with DCM. The organic phases were combined, washed with concentrated brine, dried over anhydrous sodium sulfate, and distilled under reduced pressure to obtain compound **36-1** (0.33 g, crude) as a brown oil. MS (ESI, m/z) 456 $[M+H]^+$.

Step 4: Synthesis of **BX20-9-036**

The synthesis route is shown in the following formula:

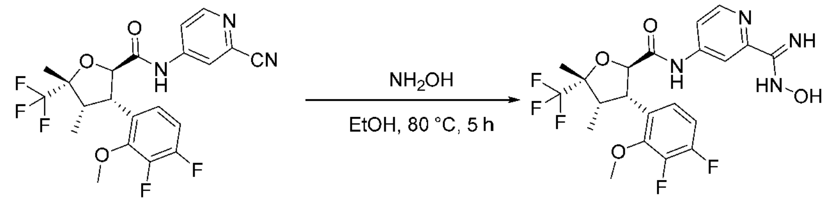


**36-1** **BX20-9-036**

Method: Compound **36-1** (0.33 g, crude, about 0.4 mmol) was dissolved in EtOH (6 mL), and $NH_2OH$ (0.11 g, 1.72 mmol, 50 wt% in water) was added. The reaction mixture was heated to 80°C and stirred for 1 hour. After the reaction was complete, the reaction mixture was concentrated under reduced pressure and purified by preparative chromatography (neutral system), followed by lyophilization to obtain **BX20-9-036** (102 mg, 49.7%) as a white solid. MS (ESI, m/z) 489 $[M+H]^+$.

$^1H$ NMR (400 MHz, DMSO-$d_6$) δ 10.07 (s, 2H), 8.42 (d, *J* = 5.6 Hz, 1H), 8.16-8.14 (m, 1H), 7.65-7.62 (m, 1H), 7.21-7.11 (m, 2H), 5.80 (s, 2H), 5.08 (d, *J* = 10.0 Hz, 1H), 4.27-4.22 (m, 1H), 3.94 (d, *J* = 2.0 Hz, 3H), 2.81-2.72 (m, 1H), 1.60 (s, 3H), 0.74-0.70 (m, 3H).

**Example 8: Synthesis of BX20-9-037**

Step 1: Synthesis of **36-1**

Steps 1 to 3 of Example 7 were referenced

Step 2: Synthesis of **37-1**

The synthesis route is shown in the following formula:

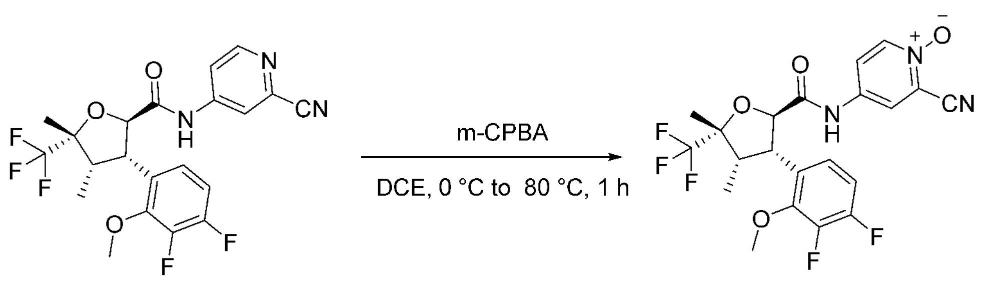


**36-1** **37-1**

Method: Compound **36-1** (220 mg, crude, about 0.4 mmol) was dissolved in DCE (5 mL) and stirred until clear. The mixture was cooled to 0°C, and m-CPBA (0.35 g, 1.72 mmol) was added. The reaction mixture was then heated to 80°C and stirred for 1 hour. After the reaction was complete, the reaction mixture was cooled to room temperature and quenched by adding to $NaHCO_3$. The mixture was extracted with DCM/MeOH, and the organic phases were combined, washed with concentrated brine, dried, and concentrated under reduced pressure to obtain **37-1** (0.26 g, crude) as a brown oil. MS (ESI, m/z) 472 $[M+H]^+$.

Step 3: Synthesis of **BX20-9-037**

The synthesis route is shown in the following formula:

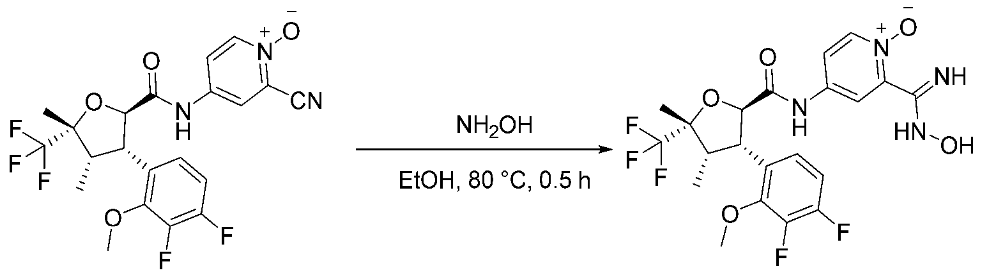


**37-1** **BX20-9-037**

Method: Compound **37-1** (0.26 g, crude) was dissolved in EtOH (6 mL), and $NH_2OH$ (0.11 g, 1.72 mmol, 50 wt% in water) was added. The reaction mixture was heated to 80°C and stirred for 0.5 hours. After the reaction was complete, the reaction mixture was directly concentrated under reduced pressure and purified by preparative chromatography (basic system), followed by lyophilization to obtain **BX20-9-037** (22 mg, 10.4%) as a light yellow solid. MS (ESI, m/z) 505 $[M+H]^+$.

$^1H$ NMR (400 MHz, DMSO-$d_6$) δ 10.72 (s, 1H), 10.15 (s, 1H), 8.21-8.15 (m, 2H), 7.71-7.67 (m, 1H), 7.21-7.11 (m, 2H), 6.78 (s, 2H), 5.07 (d, *J* = 10.0 Hz, 1H), 4.26-4.20 (m, 1H), 3.94 (d, *J* = 2.0 Hz, 3H), 2.80-2.71 (m, 1H), 1.60 (s, 3H), 0.74-0.70 (m, 3H).

**Example 9: Synthesis of BX20-9-038**

Step 1: Synthesis of intermediate S8

Steps 1 to 9 of Example 1 were referenced

Step 2: Synthesis of intermediate **38-1**

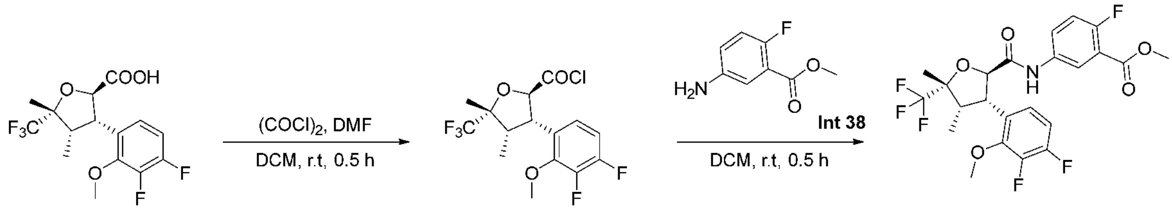


**S8** **S9** **38-1**

Method: **S8** (150 mg, 0.42 mmol) was dissolved in DCM (3 mL), followed by the addition of one drop of DMF. Oxalyl chloride (160 mg, 1.26 mmol) was added dropwise at 0°C, and the reaction mixture was stirred at room temperature for 0.5 hours. After the reaction was complete, the reaction mixture was concentrated under reduced pressure to obtain crude **S9** (170 mg, crude), which was used directly in the next step. **Int 38** (79 mg, 0.466 mmol) was dissolved in DCM (2 mL), and TEA (91 mg, 0.9 mmol) was added. A solution of **S9** (170 mg, crude) in DCM (2 mL) was then added dropwise, and the mixture was stirred at room temperature for 0.5 hours. After the reaction was complete, water was added, and the mixture was extracted with DCM, washed with brine, and concentrated under reduced pressure to obtain **38-1** (197 mg, crude) as a yellow oily liquid. MS (ESI, m/z) 506 $[M+H]^+$.

Step 3: Synthesis of intermediate **BX20-9-038**

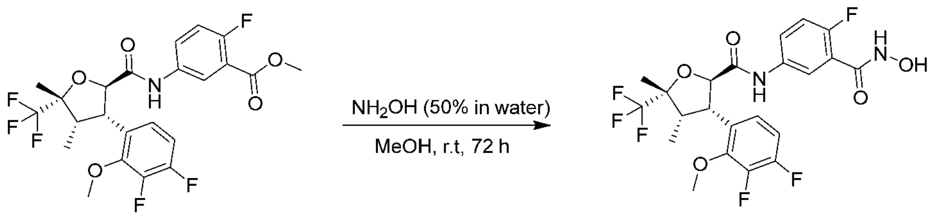


38-1 BX20-9-038

Method: Compound **38-1** (180 mg, 0.37 mmol) was dissolved in MeOH (2 mL), and $NH_2OH$ (100 mg, 1.5 mmol, 50 wt% in water) was added dropwise. The reaction mixture was stirred at room temperature for 72 hours. After the reaction was complete, the reaction mixture was concentrated under reduced pressure and purified by preparative chromatography (formic acid system), followed by lyophilization to obtain **BX20-9-038** (62 mg, yield: 34.4%) as a light yellow solid powder. MS (ESI, m/z) 507 $[M+H]^+$.

$^1H$ NMR (400MHz, DMSO-$d_6$) $\delta$ = 10.95 (brs, 1H), 10.41 (s, 1H), 9.26 (brs, 1H), 7.84-7.81 (m, 1H), 7.74-7.69 (m, 1H), 7.26-7.12 (m, 3H), 5.05 (d, $J$ = 10.4 Hz, 1H), 4.23 (dd, $J$ = 10.4 Hz, $J$ = 7.6 Hz, 1H), 3.95 (d, $J$ = 2.0 Hz, 3H), 2.79-2.72 (m, 1H), 1.60 (s, 3H), 0.74-0.70 (m, 3H).

**Example 10: Synthesis of BX20-9-039**

Step 1: Synthesis of intermediate S7

Intermediate S7 was synthesized with reference to Steps 1 to 8 of Example 1.

Step 2: Synthesis of intermediate **39-1**

The synthesis route is shown in the following formula:

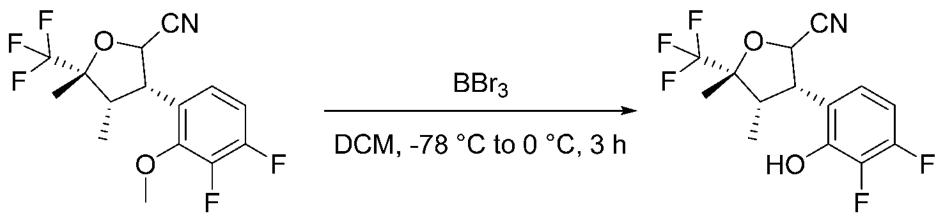


**S7** **39-1**

Method: **S7** (1.1 g, 3.28 mmol) was dissolved in DCM (15 mL), and the system was purged with $N_2$. The mixture was cooled to -78°C, and $BBr_3$ (2.1 mL, 21.32 mmol) was added dropwise slowly. After the dropwise addition was complete, the reaction mixture was warmed to 0°C and stirred for 3 hours. After the reaction was complete, the reaction mixture was slowly added dropwise to ice water, extracted with DCM, washed with saturated aqueous NaCl solution, dried over anhydrous $Na_2SO_4$, and concentrated under reduced pressure to obtain compound **39-1** (1.0 g, crude) as a yellow oil. MS (ESI, m/z) 322 $[M+H]^+$.

Step 3: Synthesis of intermediate **39-2**

The synthesis route is shown in the following formula:

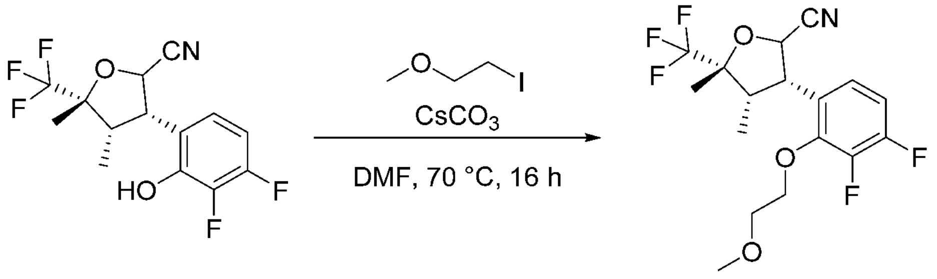


39-1 39-2

Method: **39-1** (500 mg, crude, about 1.5 mmol) was dissolved in DMF (5 mL), followed by the addition of $Cs_2CO_3$ (2.1 mL, 21.32 mmol) and 1-iodo-2-methoxyethane (1.15 g, 6.2 mmol). The reaction mixture was heated to 70°C and stirred for 16 hours. After the reaction was complete, the reaction mixture was quenched with water, extracted with EA, washed with saturated aqueous NaCl solution, dried over anhydrous $Na_2SO_4$, and concentrated under reduced pressure to obtain compound **39-2** (450 mg, crude) as a yellow oil. MS (ESI, m/z) 380 $[M+H]^+$.

Step 4: Synthesis of intermediate **39-3**

The synthesis route is shown in the following formula:

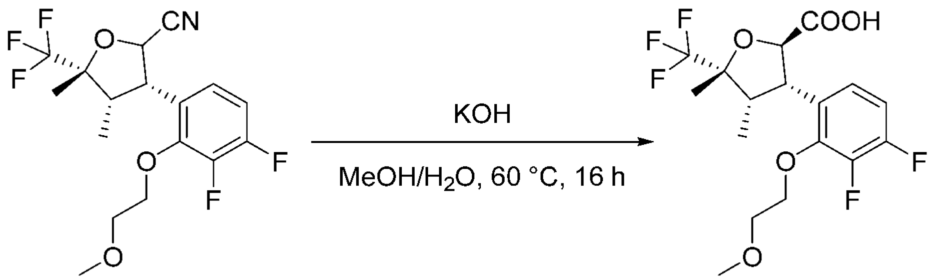


39-2 39-3

Method: **39-2** (450 mg, crude, 1.18 mmol) was dissolved in MeOH (7.5 mL), and an aqueous solution of KOH (462 mg, 8.26 mmol, dissolved in 1.5 mL water) was added. The reaction mixture was heated to 60°C and stirred for 16 hours. After the reaction was complete, the reaction mixture was concentrated under reduced pressure, diluted with water, and extracted with MTBE. The aqueous phase was adjusted to pH < 3 by dropwise addition of 1 N HCl, extracted with EA, washed with saturated aqueous NaCl solution, dried over anhydrous sodium sulfate, and concentrated under reduced pressure to obtain compound **39-3** (430 mg, crude) as a yellow oil. MS (ESI, m/z) 399 $[M+H]^+$.

Step 5: Synthesis of intermediate **39-4**

The synthesis route is shown in the following formula:

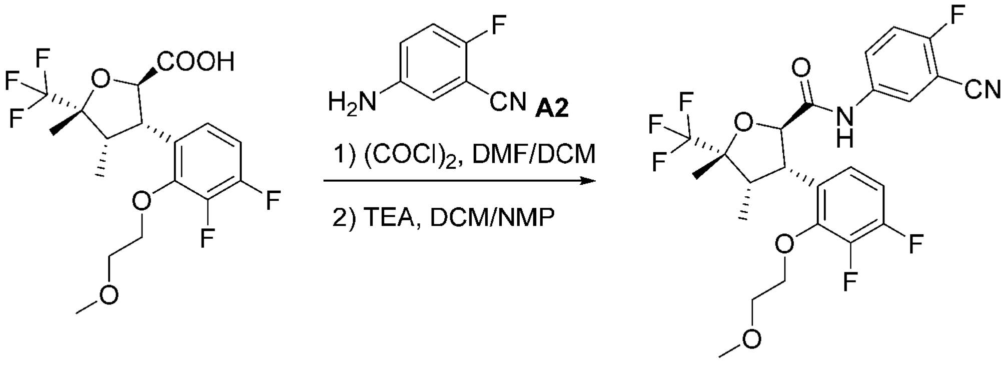


39-3 39-4

Method: **39-3** (160 mg, crude, 0.402 mmol) was dissolved in DCM (4 mL), followed by the addition of 1 drop of DMF. The mixture was cooled to 0°C, and oxalyl chloride (0.1 mL, 1.206 mmol) was added dropwise slowly. The reaction mixture was stirred at room temperature for 0.5 hours. After the reaction was complete, the mixture was concentrated under reduced pressure, redissolved in DCM (4 mL), and slowly added dropwise to a solution of 5-amino-2-fluorobenzonitrile (55 mg, 0.402 mmol) and TEA (122 mg, 1.206 mmol) in DCM/NMP (4:1, 5 mL). The reaction mixture was stirred at room temperature for 0.5 hours. After the reaction was complete, the reaction mixture was concentrated under reduced pressure, redissolved in EA, quenched with water, and extracted with EA. The organic phases were combined, washed once with water and once with saturated aqueous NaCl solution, dried over anhydrous $Na_2SO_4$, and concentrated under reduced pressure to obtain compound **39-4** (207 mg, crude) as a yellow oil. MS (ESI, m/z) 517 $[M+H]^+$.

Step 6: Synthesis of **BX20-9-039**

The synthesis route is shown in the following formula:

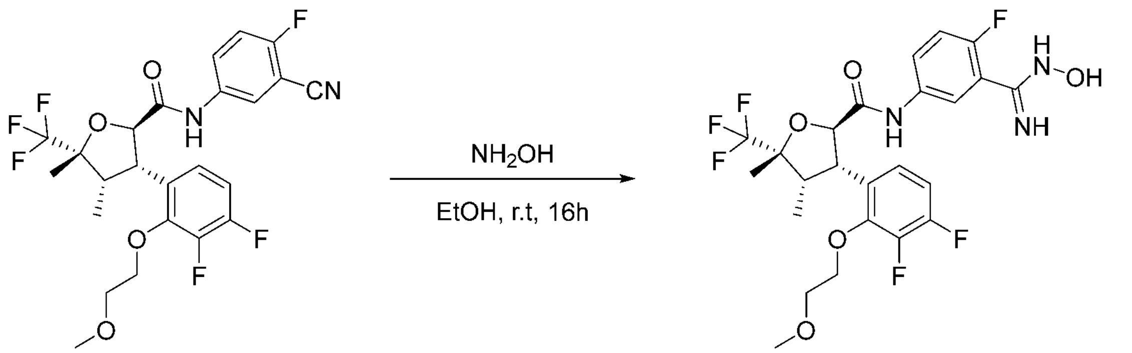


**39-4** **BX20-9-039**

Method: Compound **39-4** (207 mg, crude, about 0.4 mmol) was dissolved in EtOH (5 mL), and $NH_2OH$ (0.1 mL, 1.608 mmol, 50% in $H_2O$) was added. The reaction mixture was heated to 80°C and stirred for 1 hour. After the reaction was complete, the reaction mixture was concentrated under reduced pressure and purified by preparative chromatography (formic acid system), followed by lyophilization to obtain **BX20-9-039** (112 mg, 51%) as a white solid. MS (ESI, m/z) 550 $[M+H]^+$.

$^1H$ NMR (400 MHz, DMSO-$d_6$) δ 10.33 (s, 1H), 9.63 (s, 1H), 7.80-7.73 (m, 1H), 7.69-7.62 (m, 1H), 7.21-7.14 (m, 3H), 5.79 (s, 2H), 5.06 (d, *J* = 10.8 Hz, 1H), 4.36-4.30 (m, 1H), 4.29-4.24 (m, 1H), 4.23-4.16 (m, 1H), 3.64-3.59 (m, 2H), 3.29 (d, *J* = 1.2 Hz, 3H), 2.79-2.71 (m, 1H), 1.60 (s, 3H), 0.69 (d, *J* = 7.2 Hz, 3H).

**Example 11: Synthesis of BX20-9-040**

Step 1: Synthesis of intermediate 39-1

Intermediate 39-1 was synthesized with reference to Steps 1 to 2 of Example 10

Step 2: Synthesis of intermediate **40-1**

The synthesis route is shown in the following formula:

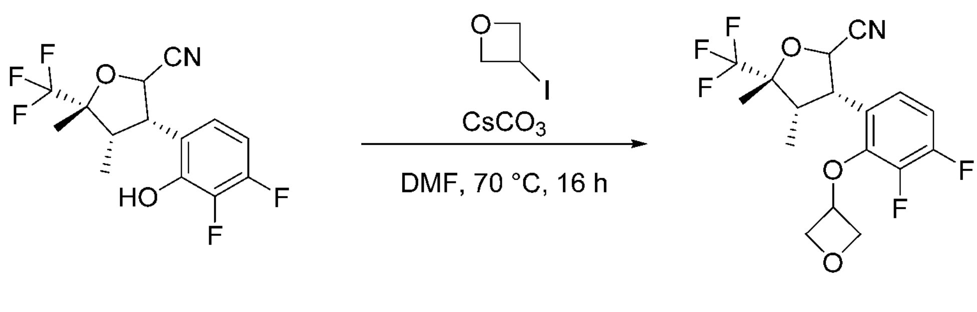


**39-1** **40-1**

Method: **39-1** (500 mg, crude, 1.55 mmol) was dissolved in DMF (5 mL), followed by the addition of $Cs_2CO_3$ (2.1 mL, 21.32 mmol) and 3-iodooxetane (1.14 g, 6.2 mmol). The reaction mixture was heated to 70°C and stirred for 16 hours. After the reaction was complete, the reaction mixture was quenched with water, extracted with EA, washed with saturated aqueous NaCl solution, dried over anhydrous $Na_2SO_4$, and concentrated under reduced pressure to obtain compound **40-1** (565 mg, crude) as a yellow oil. MS (ESI, m/z) 378 $[M+H]^+$.

Step 3: Synthesis of intermediate **40-2**

The synthesis route is shown in the following formula:

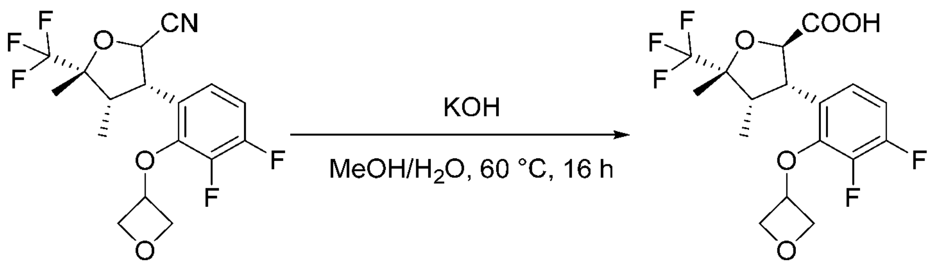


**40-1** **40-2**

Method: **40-1** (565 mg, crude, 1.5 mmol) was dissolved in MeOH (7.5 mL), and an aqueous solution of KOH (587 mg, 10.5 mmol, dissolved in 1.5 mL water) was added. The reaction mixture was heated to 60°C and stirred for 16 hours. After the reaction was complete, the reaction mixture was concentrated under reduced pressure, diluted with water, and extracted with MTBE. The aqueous phase was adjusted to pH < 3 by dropwise addition of 1 N HCl, extracted with EA, washed with saturated aqueous NaCl solution, dried over anhydrous sodium sulfate, and concentrated under reduced pressure to obtain compound **40-2** (330 mg, crude) as a yellow oil. MS (ESI, m/z) 397 $[M+H]^+$.

Step 4: Synthesis of intermediate **40-3**

The synthesis route is shown in the following formula:

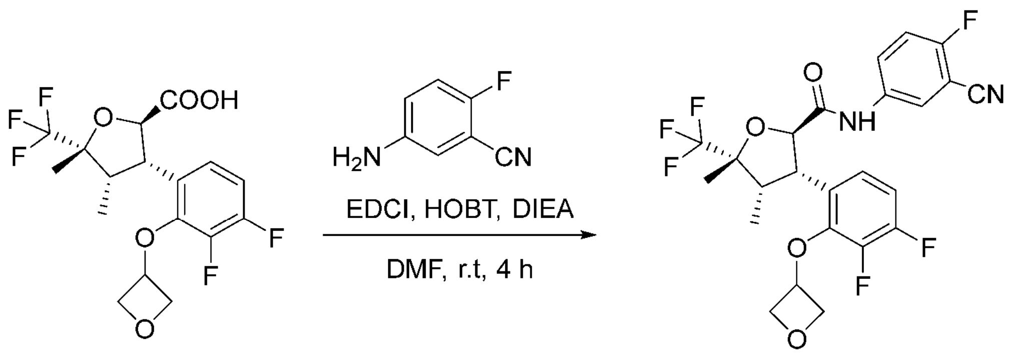


**40-2** **40-3**

Method: **40-2** (330 mg, crude, 0.83 mmol) was dissolved in DMF (6 mL), followed by the addition of EDCI (318 mg, 1.66 mmol), HOBT (135 mg, 1.0 mmol), and DIEA (321 mg, 2.4 mmol). After stirring at room temperature for 10 minutes, 5-amino-2-fluorobenzonitrile (79 mg, 0.58 mmol) was added, and the reaction mixture was stirred at room temperature for an additional 4 hours. After the reaction was complete, the reaction mixture was quenched with water, extracted with EA, washed once with water and once with saturated aqueous NaCl solution, dried over anhydrous $Na_2SO_4$, and concentrated under reduced pressure to obtain compound **40-3** (400 mg, crude) as a yellow oil. MS (ESI, m/z) 515 $[M+H]^+$.

Step 5: Synthesis of **BX20-9-040**

The synthesis route is shown in the following formula:

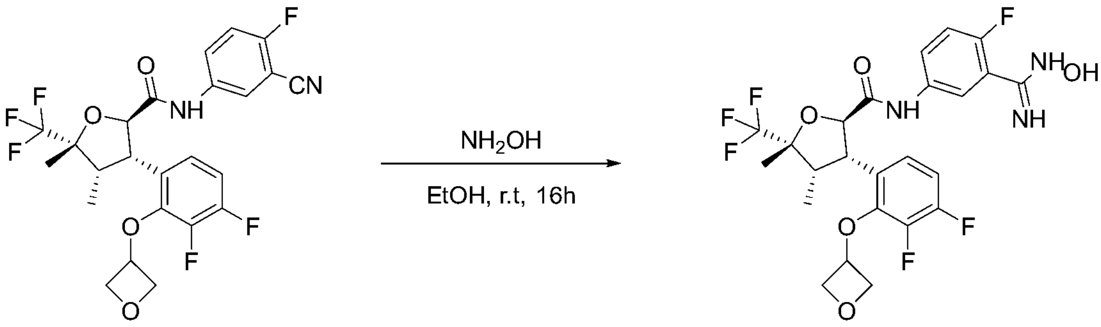


40-3

BX20-9-040

Method: Compound **40-3** (400 mg, crude, 0.78 mmol) was dissolved in EtOH (5 mL), and $NH_2OH$ (0.2 mL, 3.12 mmol, 50% in $H_2O$) was added. The reaction mixture was heated to 80°C and stirred for 1 hour. After the reaction was complete, the reaction mixture was concentrated under reduced pressure and purified by preparative chromatography (formic acid system), followed by lyophilization to obtain **BX20-9-040** (45 mg, 11%) as a white solid. MS (ESI, m/z) 548 $[M+H]^+$.

$^1H$ NMR (400 MHz, DMSO-$d_6$) $\delta$ 10.34 (s, 1H), 9.65 (s, 1H), 7.76 (d, $J$ = 6.4 Hz, 1H), 7.69-7.62 (m, 1H), 7.22-7.14 (m, 3H), 5.33-5.25 (m, 1H), 5.07 (d, $J$ = 10.4 Hz, 3H), 4.90-4.82 (m, 2H), 4.73-4.66 (m, 2H), 4.29-4.22 (m, 1H), 2.81-2.73 (m, 1H), 1.60 (s, 3H), 0.72 (d, $J$ = 7.2 Hz, 3H).

**Example 12: Synthesis of BX20-9-046**

Step 1: Synthesis of **S9**

S9 was synthesized with reference to Steps 1 to 2 of Example 7.

Step 2: Synthesis of **46-1**

The synthesis route is shown in the following formula:

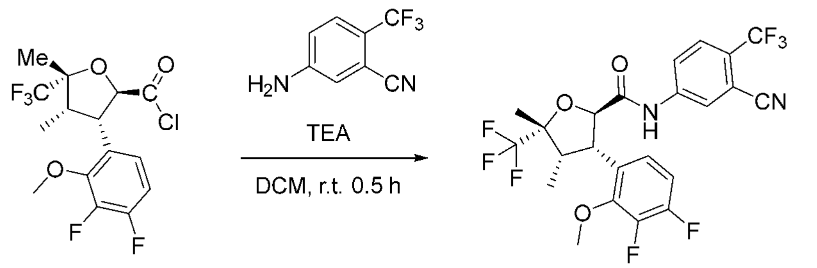


S9

46-1

Method: Compound 5-amino-2-(trifluoromethyl)benzonitrile (97 mg, 0.50 mmol) was dissolved in NMP (2 mL), followed by the addition of TEA (85 mg, 0.84 mmol). **S9** (crude, diluted with 5 mL DCM, about 0.4 mmol) was then added slowly, and the mixture was stirred at room temperature for 0.5 hours. After the reaction was complete, the reaction mixture was quenched with saturated ammonium chloride solution, extracted with DCM, and the organic phases were combined, washed with concentrated brine, dried, and concentrated under reduced pressure to obtain compound **46-1** (0.28 g, crude) as a brown oil. MS (ESI, m/z) 523 $[M+H]^+$.

Step 3: Synthesis of **BX20-9-046**

The synthesis route is shown in the following formula:

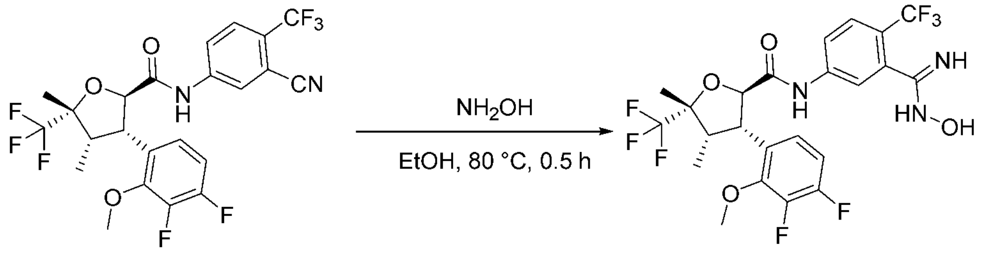


46-1 BX20-9-046

Method: Compound **46-1** (0.28 g) was dissolved in EtOH (6 mL), followed by the addition of $NH_2OH$ (0.11 g, 1.68 mmol, 50 wt% in water). The reaction mixture was heated to 80°C and stirred for 0.5 hours. After the reaction was complete, the reaction mixture was directly concentrated under reduced pressure, purified by preparative chromatography, and lyophilized to obtain **BX20-9-046** (71 mg, 30.5%) as a white solid. MS (ESI, m/z) 556 $[M+H]^+$.

$^1H$ NMR (400 MHz, DMSO-$d_6$) $\delta$ 10.62 (d, $J$ = 4.4 Hz, 1H), 9.59 (s, 1H), 7.86-7.80 (m, 2H), 7.70 (d, $J$ = 8.4 Hz, 1H), 7.22-7.11 (m, 2H), 5.88 (s, 2H), 5.12-5.07 (m, 1H), 4.28-4.21 (m, 1H), 3.95 (d, $J$ = 2.0 Hz, 3H), 2.81-2.72 (m, 1H), 1.60 (s, 3H), 0.76-0.70 (m, 3H).

## Example 13: Synthesis of BX20-9-047

Step 1: Synthesis of S8

S8 was synthesized with reference to Steps 1 to 9 of Example 1.

Step 2: Synthesis of **47-1**

The synthesis route is shown in the following formula:

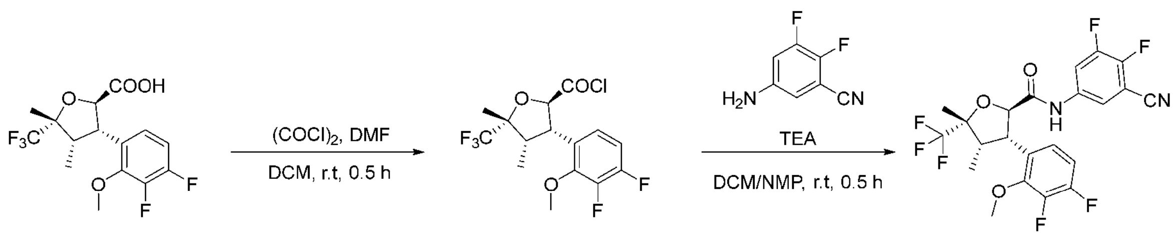


S8 S9 47-1

Method: **S8** (150 mg, 0.42 mmol) was dissolved in DCM (3 mL), followed by the addition of one drop of DMF. Oxalyl chloride (160 mg, 1.26 mmol) was added dropwise at 0°C, and the reaction mixture was stirred at room temperature for 0.5 hours. After the reaction was complete, the reaction mixture was concentrated under reduced pressure to obtain crude **S9** (180 mg, crude), which was used directly in the next step. Compound 5-amino-2,3-difluorobenzonitrile (78 mg, 0.50 mmol) was dissolved in NMP (2 mL), followed by the addition of TEA (85 mg, 0.84 mmol). **S9** (crude, diluted with 5 mL DCM, about 0.42 mmol) was then added slowly, and the reaction mixture was stirred at room temperature for 0.5 hours. After the reaction was complete, the reaction mixture was quenched with saturated ammonium chloride solution, extracted with EA, and the organic phases were combined, washed with concentrated brine, dried, and concentrated under reduced pressure to obtain compound **47-1** (0.17 g, crude) as a brown oil. MS (ESI, m/z) 491 $[M+H]^+$.

Step 3: Synthesis of **BX20-9-047**

The synthesis route is shown in the following formula:

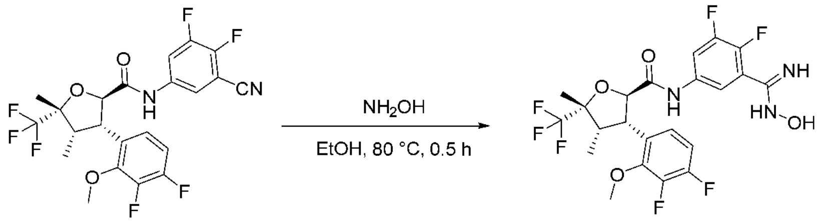


**47-1** **BX20-9-047**

Method: Compound **47-1** (crude, about 0.42 mmol) was dissolved in EtOH (6 mL), followed by the addition of $NH_2OH$ (0.11 g, 1.68 mmol, 50 wt% in water). The reaction mixture was heated to 80°C and stirred for 0.5 hours. After the reaction was complete, the reaction mixture was directly concentrated under reduced pressure and purified by preparative chromatography (formic acid system) to obtain **BX20-9-047** (52 mg, 23.6%) as a white solid. MS (ESI, m/z) 524 $[M+H]^+$.

$^1H$ NMR (400 MHz, DMSO-$d_6$) δ = 9.87 (s, 1H), 9.76 (s, 1H), 7.97-7.92 (m, 1H), 7.48-7.40 (m, 1H), 7.36-7.31 (m, 1H), 7.20-7.12 (m, 1H), 6.28 (s, 2H), 5.19 (d, *J* = 11.2 Hz, 1H), 4.06-3.99 (m, 1H), 3.90 (d, *J* = 2.0 Hz, 3H), 2.74-2.64 (m, 1H), 1.65 (s, 3H), 0.74-0.69 (m, 3H).

**Example 14: Synthesis of BX20-9-048**

Step 2: Synthesis of **48-2**

The synthesis route is shown in the following formula:

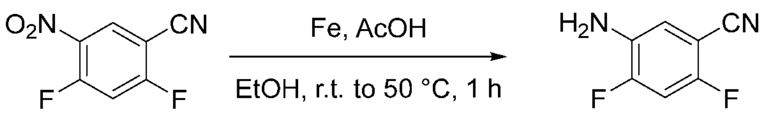


**48-1** **48-2**

Method: Compound **48-1** (0.50 g, 2.7 mmol) was dissolved in EtOH (4 mL), followed by the addition of AcOH (0.97 g, 16.2 mmol) and reduced iron powder (0.76 g, 13.5 mmol). The reaction mixture was stirred at room temperature for 0.5 hours, then heated to 50°C and stirred for an additional 0.5 hours. After the reaction was complete, the reaction mixture was directly filtered, added with water, and extracted with EA. The organic phases were combined, washed with concentrated brine, dried, and concentrated under reduced pressure to obtain **48-2** (0.40 g, 96.3%). MS (ESI, m/z) 155 $[M+H]^+$.

Step 2: Synthesis of **48-3**

The synthesis route is shown in the following formula:

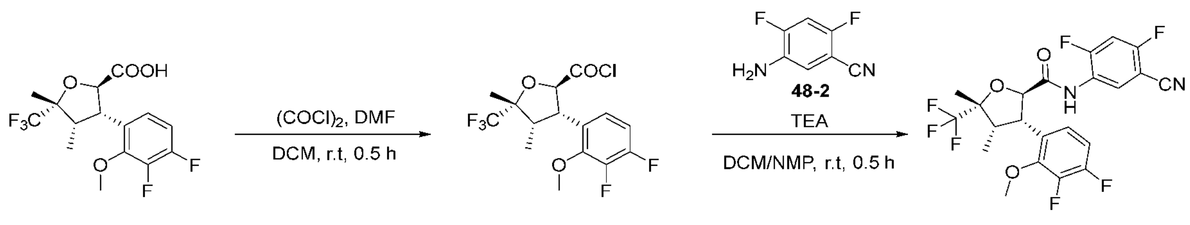


**S8** **S9** **48-3**

Method: **S8** (150 mg, 0.42 mmol) was dissolved in DCM (3 mL), followed by the addition of one drop of DMF. Oxalyl chloride (160 mg, 1.26 mmol) was added dropwise at 0°C, and the reaction mixture was stirred at room temperature for 0.5 hours. After the reaction was complete, the reaction mixture was concentrated under reduced pressure to obtain crude **S9** (180 mg, crude), which was used directly in the next step. 5-Amino-2,4-difluorobenzonitrile (78 mg, 0.50 mmol) was dissolved in NMP (2 mL), followed by the addition of TEA (85 mg, 0.84 mmol). **S9** (crude, diluted with 5 mL DCM, 0.42 mmol) was then added slowly, and the reaction mixture was stirred at room temperature for 0.5 hours. After the reaction was complete, the reaction mixture was quenched with saturated ammonium chloride solution, extracted with DCM, and

the organic phases were combined, washed with concentrated brine, dried, and concentrated under reduced pressure to obtain compound **48-3** (0.15 g, crude) as a brown oil. MS (ESI, m/z) 491 $[M+H]^+$.

Step 3: Synthesis of **BX20-9-048**

The synthesis route is shown in the following formula:

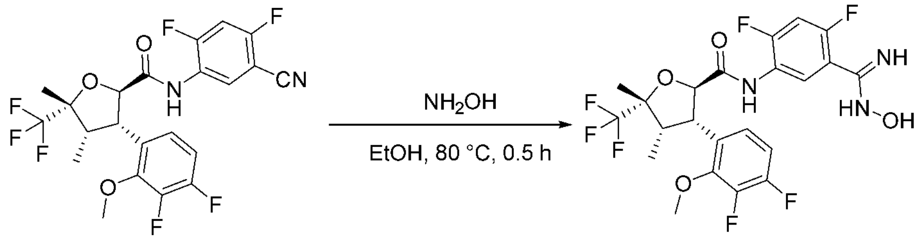


**48-3** **BX20-9-048**

Method: Compound **48-3** (0.15 g, crude, about 0.42 mmol) was dissolved in EtOH (6 mL), followed by the addition of $NH_2OH$ (0.11 g, 1.72 mmol, 50 wt% in water). The reaction mixture was heated to 80°C and stirred for 0.5 hours. After the reaction was complete, the reaction mixture was directly concentrated under reduced pressure and purified by preparative chromatography (neutral system) to obtain **BX20-9-048** (56 mg, 27.2%) as a white solid. MS (ESI, m/z) 524 $[M+H]^+$.

$^1H$ NMR (400 MHz, DMSO-$d_6$) δ 9.96 (s, 1H), 9.64 (s, 1H), 7.85-7.78 (m, 1H), 7.44-7.33 (m, 1H), 7.24-7.12 (m, 2H), 5.81 (s, 2H), 5.19 (d, *J* = 10.4 Hz, 1H), 4.24-4.17 (m, 1H), 3.94 (d, *J* = 2.0 Hz, 3H), 2.83-2.66 (m, 1H), 1.60 (s, 3H), 0.74-0.70 (m, 3H).

**Example 15: Synthesis of BX20-9-049**

Step 1: Synthesis of S8

S8 was synthesized with reference to Steps 1 to 9 of Example 1.

Step 2: Synthesis of **49-1**

The synthesis route is shown in the following formula:

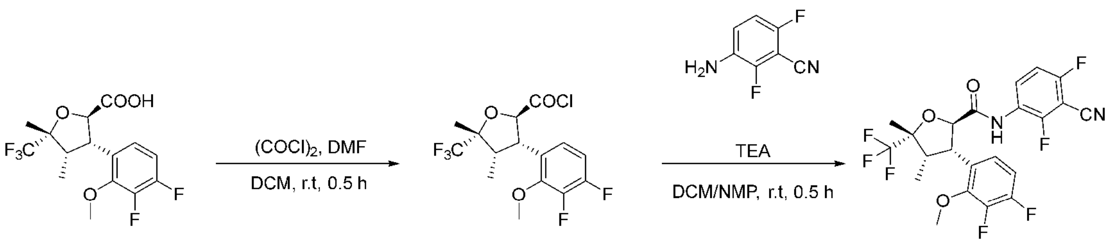


**S8** **S9** **49-1**

Method: **S8** (150 mg, 0.42 mmol) was dissolved in DCM (3 mL), followed by the addition of one drop of DMF. Oxalyl chloride (160 mg, 1.26 mmol) was added dropwise at 0°C, and the reaction mixture was stirred at room temperature for 0.5 hours. After the reaction was complete, the reaction mixture was concentrated under reduced pressure to obtain crude **S9** (180 mg, crude), which was used directly in the next step. Compound 3-amino-2,6-difluorobenzonitrile (78 mg, 0.50 mmol) was dissolved in NMP (2 mL), followed by the addition of TEA (85 mg, 0.84 mmol). **S9** (crude, diluted with 5 mL DCM, about 0.42 mmol) was then added slowly, and the reaction mixture was stirred at room temperature for 0.5 hours. After the reaction was complete, the reaction mixture was quenched with saturated ammonium chloride solution, extracted with EA, and the organic phases were combined, washed with concentrated brine, dried, and concentrated under reduced pressure to obtain compound **49-1** (0.13 g, crude) as a brown oil. MS (ESI, m/z) 491 $[M+H]^+$.

Step 3: Synthesis of **BX20-9-049**

The synthesis route is shown in the following formula:

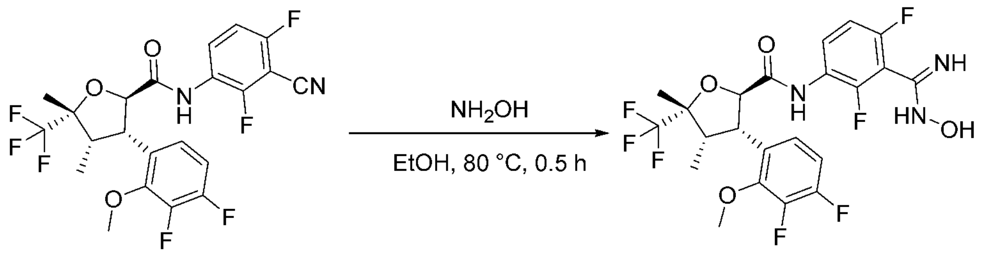


**49-1** **BX20-9-049**

Method: Compound **49-1** (0.13 g, crude, about 0.42 mmol) was dissolved in EtOH (6 mL), followed by the addition of $NH_2OH$ (0.11 g, 1.68 mmol, 50 wt% in water). The reaction mixture was heated to 80°C and stirred for 0.5 hours. After the reaction was complete, the reaction mixture was directly concentrated under reduced pressure and purified by preparative chromatography (formic acid system) to obtain **BX20-9-049** (48 mg, 21.9%) as a white solid. MS (ESI, m/z) 524 $[M+H]^+$.

$^1H$ NMR (400 MHz, DMSO-$d_6$) δ 9.98 (s, 1H), 9.60 (s, 1H), 7.79-7.69 (m, 1H), 7.24-7.14 (m, 2H), 7.12-7.05 (m, 1H), 5.97 (s, 2H), 5.21 (d, *J* = 10.4 Hz, 1H), 4.23-4.16 (m, 1H), 3.94 (d, *J* = 2.0 Hz, 3H), 2.80-2.69 (m, 1H), 1.60 (s, 3H), 0.74-0.70 (m, 3H).

Example 16: Synthesis of **BX20-9-055**

Step 1: Synthesis of BX20-9-029

The synthesis of BX20-9-029 was completed with reference to Steps 1 to 3 of Example 3

Step 2: Synthesis of intermediate **55-1**

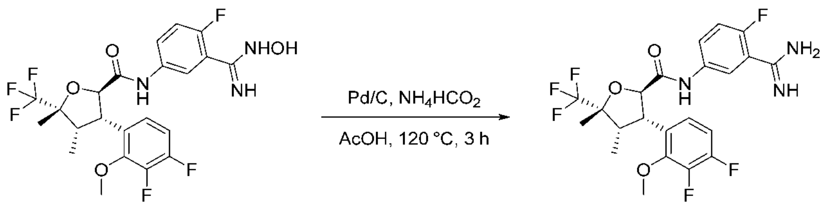


**BX20-9-029** **55-1**

Method: **BX20-9-029** (100 mg, 0.2 mmol) was dissolved in AcOH (5 mL), followed by the addition of Pd/C (100 mg, 50% wt water) and ammonium formate (126 mg, 2 mmol). The reaction system was purged with nitrogen and then heated to 120°C with stirring under reflux for 3 hours. After the reaction was complete, the reaction mixture was filtered, and the filtrate was concentrated under reduced pressure to obtain **55-1** (90 mg, crude) as a yellow oily liquid. MS (ESI, m/z) 490 $[M+H]^+$.

Step 3: Synthesis of **BX20-9-055**

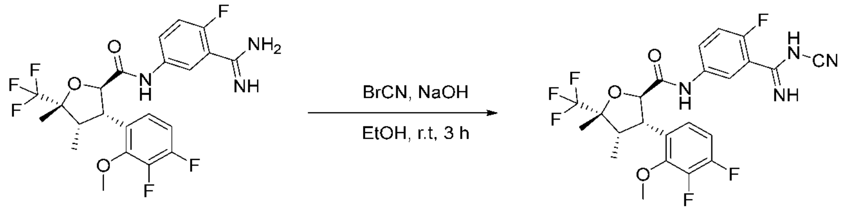


55-1 BX20-9-055

Method: **55-1** (90 mg, crude) was dissolved in EtOH (3 mL), followed by the addition of NaOH (80 mg, 2 mmol) and cyanogen bromide (106 mg, 1 mmol). The reaction was stirred at room temperature for 3 hours. After the reaction was complete, the reaction mixture was quenched with water, extracted three times with EA, and the organic phases were combined, washed with brine, dried over anhydrous sodium sulfate, concentrated under reduced pressure, and purified by preparative chromatography to obtain **BX20-9-055** (11 mg, yield: 11%) as a white solid. MS (ESI, m/z) 515 $[M+H]^+$.

$^1H$ NMR (400 MHz, DMSO-$d_6$) $\delta$ = 10.44 (brs, 1H), 7.83 (brs, 1H),7.77-7.73 (m, 1H), 7.32 (t, $J$ = 9.2 Hz, 1H), 7.17-7.09 (m, 2H), 5.04 (d, $J$ = 10.4 Hz, 1H), 4.21 (dd, $J$ = 10.4 Hz, 8.4 Hz, 1H), 3.92 (d, $J$ = 2.0 Hz, 3H), 2.77-2.69 (m, 1H), 1.57 (s, 3H), 0.70 (d, $J$ = 6.8 Hz, 3H).

**Reference compound**:

The reference compound (compound 7) was synthesized with reference to the synthesis method described in Example 3 of patent CN114945566A.

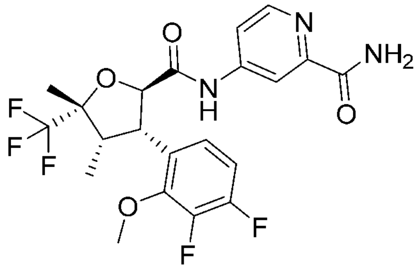

$^1H$ NMR (400 MHz, $CD_3OD$) $\delta$ = 8.48 (d, $J$ = 5.6 Hz, 1H), 8.25 (d, $J$ = 2.0 Hz, 1H), 7.89 (dd, $J$ = 2.0 Hz, 5.2 Hz, 1H), 7.13-7.09(m, 1H), 7.00-6.94 (m, 1H), 5.08 (d, $J$ = 10.4 Hz, 1H), 4.34-4.30 (m, 1H), 3.99 (d, $J$ = 2.4 Hz, 3H), 2.83-2.75 (m, 1H), 1.65 (s, 3H), 0.82-0.80 (m, 3H)

**Biological test evaluation**

The present disclosure was further illustrated and described in combination with the following test examples

Test Example 1: Blocking activity of the compounds of the present disclosure against sodium channel 1.8 (Nav1.8)

1. Experimental objective: The effects of the compounds on the current of the voltage-gated sodium channel (NaV) 1.8 subtype were detected by patch-clamp technique

2. Experimental materials and instruments

2.1 Cell line: CHO cell line that stably expressed the Nav1.8 sodium channel, Nav1.8 cells were constructed in-house by the laboratory of Beijing ICE Bioscience Co., Ltd., gene information: Sodium channel, voltage-gated, type 8, alpha (SCN10A), cDNA strictly similar to GenBank accession number: NM_006514

2.2 Compounds: Dissolved in DMSO.

3. Experimental methods

3.1 Cell culture

(1) Maintenance medium: The cells were cultured in HAM'S/F-12 medium containing 10% fetal bovine serum and 10 $\mu$g/mL Blasticidin, 200 $\mu$g/mL Hygromycin B, and 100 $\mu$g/mL Zeocin; the culture temperature was 37°C with a carbon dioxide concentration of 5%.

(2) Cell passage: The old medium was removed, and the cells were washed once with PBS, then 1 mL of 0.25%-Trypsin-EDTA solution was added and incubated at 37°C for approximately 1.5 min. When the cells were

detached from the bottom of the dish, about 5 mL of preheated complete medium at 37°C was added. The cell suspension was gently pipetted up and down to separate the aggregated cells. The cell suspension was transferred to a sterile centrifuge tube and centrifuged at 1000 rpm for 5 min to collect the cells. For amplification or maintenance culture, the cells were seeded into 6 cm cell culture dishes, with a seeding density of $2.5 \times 10^5$ cells per dish (final volume: 5 mL).

(3) To maintain the electrophysiological activity of the cells, the cell density was required not to exceed 80%.

(4) For patch-clamp detection, the cells were dissociated with 0.25%-Trypsin-EDTA before the experiment, $6.5 \times 10^3$ cells were plated onto coverslips, cultured in a 24-well plate (final volume: 500 μL), and tested after 18 hours.

3.2 Patch-clamp detection

(1) After the whole-cell configuration was formed, the cell voltage was clamped at - 120 mV. First, the voltage was stepped from -130 mV to -10 mV in 10 mV increments for 5 s, and then a 0 mV depolarizing pulse was applied to obtain the half-inactivation voltage (Vhalf). The resting state and half-inactivated state of the sodium current were detected by using a double-pulse protocol. First, the first depolarizing pulse (TP1) was applied to 0 mV for 50 ms to detect the sodium current in the resting state. Then, the voltage was adjusted to Vhalf and maintained for 5 s, followed by restoration of the voltage to -120 mV and maintenance for 20 ms, after which the second depolarizing pulse (TP2) was applied to 0 mV for 50 ms to detect the sodium current in the half-inactivated state. Finally, the voltage was restored to the clamping voltage of -120 mV. Data were repeatedly collected every 20 ms to observe the effect of the drug on the peak values of sodium currents in the two different states. Experimental data were collected by the EPC 10 amplifier (HEKA) and stored in the PatchMaster (HEKA) software.

(2) During patch clamp operation, capillary glass tubes were first pulled into recording electrodes using a microelectrode puller, then the electrodes filled with intracellular solution were installed into the microelectrode holder. Under an inverted microscope, the micromanipulator was used to bring the recording electrode into contact with the cell, and negative pressure suction was applied to form a GΩ seal. At this time, fast capacitance compensation was performed, and then negative pressure was continuously applied to rupture the cell membrane and form the whole-cell recording mode. Finally, slow capacitance compensation was performed, and related parameters were recorded. Leak compensation was not applied.

(3) When the sodium current of whole-cell recording became stable, drug administration was started, and each drug concentration was applied for 5 min (or until the current became stable) before the next concentration was tested. The coverslip coated with cells was placed in the recording bath under an inverted microscope, and the blank control external solution and the test compound working solution were perfused through the recording bath in sequence from low concentration to high concentration by gravity, thereby acting on the cells, while a peristaltic pump was used for liquid exchange during recording. The current detected in the external solution without compounds for each cell was used as its own control group. Each concentration was independently tested twice. All electrophysiological experiments were conducted at room temperature.

3.3 Data analysis

First, the current after the effect of each drug concentration and the blank control current were normalized, then the inhibition rate corresponding to each drug concentration was calculated, that is, (1-Icompound /Icontrol), and the mean, standard deviation (SD), and standard error (SE) of the inhibition rate for each concentration were calculated, and the data were expressed as mean ± SE.

4. Experimental results

Table 1: Blocking rate of the compounds of the present disclosure against NaV1.8

| Compound No. | Inhibition rate (%) |
|---|---|
| Reference compound | 94.49 |
| Example 1 | 80.08 |
| Example 2 | 76.36 |
| Example 3 | 98.33 |
| Example 5 | 50.04 |

(continued)

| Compound No. | Inhibition rate (%) |
|---|---|
| Example 6 | 71.63 |
| Example 7 | 95.55 |
| Example 8 | 90.96 |
| Example 9 | 97.24 |
| Example 10 | 99.65 |
| Example 11 | 87.35 |
| Example 12 | 76.58 |
| Example 13 | 89.85 |
| Example 14 | 84.01 |
| Example 15 | 56.23 |
| Example 16 | 95.94 |

It was observed that the compounds of the present disclosure had obvious blocking effects on the NaV1.8 channel activity.

**Test Example 2:** Blocking activity ($IC_{50}$) of the compound of the present disclosure against sodium channel 1.8 (Nav1.8)

1. **Research objective**

The manual patch-clamp technique was applied to evaluate whether the test compound had a potential inhibitory effect against the voltage-gated sodium channel hNav1.8. This experiment was performed by detecting the influence of 5 concentrations of the compound or a single point or two concentration points on the hNav1.8 channel current, the dose-response curve of the compound was obtained, and the $IC_{50}$ was calculated. This experiment was conducted to include two parallel sample determinations.

2. Experimental methods

2.1 Experimental materials

1) Cells: The HEK293 cell line that stably expressed the hNav1.8/β3 ion channel was prepared in-house by the Biology Department of Pharmaron (Beijing) Co., Ltd. (The lentiviral vectors that respectively expressed human Nav1.8 and β3 were transduced into HEK293, and it was prepared after screening. Specific reference: Proc Natl Acad Sci U S A. 2022 Jul 26;119(30):e2208211119. doi: 10.1073/pnas.2208211119. and the Cell Lines / BSYS CHO NaV1.8/β3 Cell Line manual.). The cell line was cultured in a medium containing 90% DMEM, 10% fetal bovine serum, 100 U/mL penicillin-streptomycin solution, 0.75 μg/mL puromycin, and 100 μg/mL hygromycin. When the cell density had grown to 40% to 80% of the bottom area of the culture dish, digestion was performed with trypsin, and passage was carried out three times per week. Before the experiment, the cells were cultured in a 6 cm culture dish at a total number of $5 \times 10^5$ and were seeded on coverslips for subsequent manual patch-clamp experiments.
2) Compound: The test compound was dissolved in DMSO and prepared as a stock solution with a final concentration of 10 or 30 mM. The stock solution was diluted with DMSO as the solvent to obtain the required intermediate solution. Before the experiment began, the gradient intermediate solutions of the test compounds were further diluted with extracellular solution at a ratio of 1:1000 to prepare a series of working solutions with different concentrations. The content of DMSO in the working solution was 0.1% (v/v). The working solutions with different concentration gradients were used to determine the potential inhibitory effect of the compounds on the hNav1.8 channel and to fit the dose-response curve and calculate the $IC_{50}$.

2.2 Experimental procedures

1) The small glass slide carrying HEK293 cells in the culture dish was placed in the perfusion chamber of the micromanipulation stage.
2) Under an Olympus IX71 or IX73 inverted microscope, a suitable cell was adjusted to the center of the field of view, the tip of the glass electrode was located using a ×10 objective lens, and it was placed at the center of the field of view. Then the electrode was lowered using a micromanipulator, while the coarse focus knob was adjusted to make the electrode slowly approach the cell.
3) When the electrode was close to the cell, observation was switched to a ×40 objective lens, and the fine adjustment of the micromanipulator was used to make the electrode gradually approach the surface of the cell.
4) Negative pressure was applied to form a seal with a resistance greater than 1 GΩ between the tip of the electrode and the cell membrane.
5) The transient capacitive current Cfast was compensated under the voltage-clamp mode. Then, brief negative pressure was repeatedly applied to rupture the membrane, and the whole-cell recording mode was finally formed.
6) Under the condition that the membrane potential was clamped at -60 mV, the slow capacitive current Cslow, cell membrane capacitance (Cm), and input membrane resistance (Ra) were respectively compensated.
7) After the cell was stabilized, the clamping voltage was changed to -80 mV, the sampling frequency was set to 20 kHz, and the filtering frequency was
10 kHz. The detection condition of the leakage current was that the clamping voltage was changed to -80 mV for a duration of 200 ms.
8) The hNav1.8 current testing method was as follows: A 20 ms depolarization command voltage was applied to depolarize the membrane potential from -80 mV to -10 mV, and then the membrane potential was repolarized to -80 mV to close the channel. The stimulation was applied once every 15 seconds. The instantaneous current peak under the depolarization voltage was the magnitude of the Nav1.8 sodium channel current.
9) The hNav1.8 current used for detecting the test compound was continuously recorded for 120 seconds before administration to evaluate the stability of the test cell in generating the hNav1.8 current. Only stable cells within the acceptable range of evaluation criteria were allowed to enter subsequent compound detection.
10) Test of the inhibitory effect of the test compound on the hNav1.8 current: First, the hNav1.8 current measured in extracellular solution containing 0.1% DMSO was used as the detection baseline. After the hNav1.8 current had remained stable for at least 5 minutes, the solution containing the test compound was perfused around the cells sequentially from low concentration to high concentration. After each perfusion had ended, approximately 5 minutes were waited to allow the compound to fully act on the cells, and the hNav1.8 current was synchronously recorded. After the recorded current had tended to stabilize, the last five hNav1.8 current values were recorded, and their average value was taken as the final current value at the specific concentration. After the compound had been tested, 5 nM reference compound was added to the same cell to completely inhibit its current, serving as the positive control for that cell. Meanwhile, the positive compound reference compound was synchronously detected with the same patch-clamp system before and after the test drug experiment was completed, to ensure the reliability and sensitivity of the entire detection system. The above testing procedures were repeated on two separate test cells (n = 2).

2.3 **Data analysis**

1) The data were required to meet the following criteria: The initial sealing resistance was greater than 1 GΩ; the rupture membrane resistance Ra was less than 15 MΩ; the leakage current under the detection voltage was less than 50% of the current value under the same condition; the Nav1.8 peak current was at least greater than 200 pA.
2) The data were analyzed according to the following steps (the data were output by PatchMaster software):

a. After perfusing the blank solvent or the compound gradient solution, 5 consecutive stable current values were obtained and averaged, respectively, as "$current_{blank}$" and "$current_{compound}$". The percentage of current inhibition was calculated using the following formula.

$$\text{Tail current inhibition rate} = \left(1 - \frac{\text{Current}_{\text{compound}} - \text{Current}_{\text{positive drug}}}{\text{Current}_{\text{blank}} - \text{Current}_{\text{positive drug}}}\right) \times 100$$

b. The dose-response curve was fitted by Graphpad Prism 8.0 software, and the $IC_{50}$ value was calculated.

The standard deviation range of the two groups of data was less than 15 (SD < 15).

3. Experimental results

Table 2: Blocking activity of the compound of the present disclosure against NaV1.8

| Compound No. | Inhibitory activity ($IC_{50}$) |
|---|---|
| Reference compound | 0.297 |
| Example 11 | 0.229 |

It was observed that the compound of the present disclosure had very strong inhibitory activity against the NaV1.8 channel, which was better than the reference compound.

Test Example 3: Selectivity test of the compounds of the present disclosure on sodium channels

1. Experimental objective: The effects of the compounds on the current of the voltage-gated sodium channel (NaV) subtypes 1.1-1.7 were detected by patch-clamp technique
2. Experimental materials and instruments

2.1 Cell lines: CHO/HEK293 cell lines stably expressing Nav1.1-1.7 sodium channels, constructed in-house by the laboratory of Beijing ICE Bioscience Co., Ltd., (Reference: Jarvis MF, Honore P, Shieh CC, Chapman M, Joshi S, Zhang XF, Kort M, Carroll W, Marron B, Atkinson R, Thomas J, Liu D, Krambis M, Liu Y, McGaraughty S, Chu K, Roeloffs R, Zhong C, Mikusa JP, Hernandez G, Gauvin D, Wade C, Zhu C, Pai M, Scanio M, Shi L, Drizin I, Gregg R, Matulenko M, Hakeem A, Gross M, Johnson M, Marsh K, Wagoner PK, Sullivan JP, Faltynek CR, Krafte DS. A-803467, a potent and selective Nav1.8 sodium channel blocker, attenuates neuropathic and inflammatory pain in the rat. Proc Natl Acad Sci U S A. 2007 May 15;104(20):8520-5. doi: 10.1073/pnas.0611364104. Epub 2007 May 2. PMID: 17483457; PMCID: PMC1895982.), gene information Nav1.1: NM_006920; Nav1.2: NM_001040142; Nav1.3: NM_006922; Nav1.4: NM_000334; Nav1.5: NM198056; Nav1.6: NM014191; Nav1.7: NM006922.
2.2 Compounds: Dissolved in DMSO, with sample concentration prepared at 30 μM.

3. Experimental methods

3.1 Cell culture

(1) Maintenance medium: CHO cells were cultured in HAM'S/F-12 medium containing 10% fetal bovine serum, 100 μg/mL Zeocin, and 10 μg/mL Blasticidin, under conditions of 37°C incubation temperature and 5% carbon dioxide concentration. HEK-293 cells were cultured in DMEM medium containing 10% fetal bovine serum and 800 μg/mL G418; the culture temperature was 37°C, and the carbon dioxide concentration was 5%.
(2) Cell passage: The old medium was removed, and the cells were washed once with PBS, then 1 mL of 0.25%-Trypsin-EDTA solution was added and incubated at 37°C for approximately 1.5 min. When the cells were detached from the bottom of the dish, about 5 mL of preheated complete medium at 37°C was added. The cell suspension was gently pipetted up and down to separate the aggregated cells. The cell suspension was transferred to a sterile centrifuge tube and centrifuged at 1000 rpm for 5 min to collect the cells. For amplification or maintenance culture, the cells were seeded into 6 cm cell culture dishes, with a seeding density of $2.5 \times 10^5$ cells per dish (final volume: 5 mL).
(3) To maintain the electrophysiological activity of the cells, the cell density was required not to exceed 80%.

(4) For patch-clamp detection, the cells were dissociated with 0.25%-Trypsin-EDTA before the experiment, $6.5 \times 10^3$ cells were plated onto coverslips, cultured in a 24-well plate (final volume: 500 μL), and tested after 18 hours.

3.2 Patch-clamp detection
Same as Test Example 1 3.2
3.3 Data analysis
Same as Test Example 1 3.3

4. Experimental results

Table 3: Blocking rate of the compounds of the present disclosure against NaV1.1-1.7 at 30 μM

| Compound No. | Nav1.1 | Nav1.2 | Nav1.3 | Nav1.4 | Nav1.5 | Nav1.6 | Nav1.7 |
|---|---|---|---|---|---|---|---|
| | Inhibition rate (%) | Inhibition rate (%) | Inhibition rate (%) | Inhibition rate (%) | Inhibition rate (%) | Inhibition rate (%) | Inhibition rate (%) |
| Reference compound | 57.73 | 53.64 | 63.36 | 83.56 | 72.21 | 42.87 | 11.12 |
| Example 10 | 28.39% | 32.75% | 28.59% | 75.37% | 51.69% | 36.04% | 20.83% |
| Example 11 | 1.96% | 16.01% | 21.72% | 8.45% | 9.11% | 19.49% | 14.75% |

Conclusion: It was observed that the compounds of the present disclosure were not significantly active against NaV1.1-1.7 channels; at the same concentration, the inhibitory activity against NaV1.1-1.6 channels was lower than that of the reference compound, showing better target selectivity, indicating that the compounds of the present disclosure had better safety.

**Test Example 4: Pharmacokinetic determination in SD rats**

1. Experimental objective

SD rats were used as test animals to study the pharmacokinetic behavior in rat plasma after oral administration of the compound example of the present disclosure at a dose of 10 mg/kg.

2. Experimental methods

2.1. Experimental drugs

The examples of the present disclosure and the reference compound were self-prepared.

2.2. Experimental animals

Male SPF-grade SD rats, with body weight (200 ± 20) g.

2.3. Preparation of test drugs

Drug preparation: The drug concentration was prepared as 1 mg/mL, and the preparation solvent was Tween80 + 0.5% MC (v/v 1:99).

2.4. Administration:

Male SPF-grade SD rats were administered by gavage after adaptive feeding for 3-4 days, with an administration dose of 10 mg/kg and an administration volume of 10 mL/kg.

2.5. Sample collection

Blood samples were collected from rats before administration (0 h) and after administration at 0.5 h, 1 h, 1.5 h, 2 h,

3 h, 4 h, 6 h, 8 h, 10 h, 12 h, and 24 h. Blood, approximately 0.2 mL, was collected by retro-orbital puncture and placed into EDTA-K2 anticoagulant tubes with labels. After blood collection, the tubes were gently and completely inverted three times to mix with the anticoagulant. The samples were then immediately placed in an ice-water bath and centrifuged at 4500 rpm for 10 min at 4°C. After centrifugation, the plasma was promptly aliquoted into EP tubes with corresponding labels and stored in a -80°C freezer.

2.6. Sample testing

After administration, 20 μL of rat plasma was taken and placed into a 96-deep-well plate pre-filled with 20 μL of internal standard working solution. Methanol (400 μL) as the precipitating agent was added, vortexed for 10 min, and centrifuged at 4000 rpm for 15 min. A supernatant of 200 μL was transferred into another 96-deep-well plate pre-filled with 200 μL of ultrapure water, vortexed for 5 min, and centrifuged at 4000 rpm for 3 min. A supernatant of 5 μL was taken for LC-MS/MS analysis of plasma drug concentration.

3. Experimental results and analysis

The main pharmacokinetic parameters were calculated by WinNonlin 7.0, and the pharmacokinetic experimental results of rats are shown in Table 4 below.

Table 4: Pharmacokinetic experimental results of rats

| No. | Half-life | Time to peak | Peak concentration | Area under the curve |
|---|---|---|---|---|
| | $t_{1/2}$ (h) | $T_{max}$ (h) | Cmax (ng/mL) | $AUC_{0-t}$ (h × ng/mL) |
| Reference compound | 1.32 ± 0.17 | 0.63 ± 0.25 | 216.43 ± 124.26 | 607.85 ± 361.59 |
| Example 10 | 1.60 ± 0.27 | 0.88 ± 0.25 | 1242.43 ± 325.92 | 4649.19 ± 1604.09 |
| Example 11 | 0.85 ± 0.07 | 0.75 ± 0.50 | 1043.30 ± 260.37 | 2076.22 ± 200.82 |

4. Experimental conclusion

It was known from the above data that, under the same administration dose, the exposure of the compounds of the present disclosure was higher than that of the reference compound, indicating that the compounds of the present disclosure exhibited better absorption characteristics.

**Test Example 5: Pharmacokinetic determination in KM mice**

1. Experimental objective

Using KM mice as test animals, the pharmacokinetic behavior of the compounds in the examples in mouse plasma after administration by gavage at a dose of 10 mg/kg or by intravenous injection at a dose of 1 mg/kg was studied.

2. Experimental methods

2.1. Experimental drugs

The examples of the present disclosure and the reference compound were self-prepared.

2.2. Experimental animals

Male SPF-grade KM mice, with body weight (20 ± 2) g.

2.3. Preparation of test drugs

Drug preparation for the intragastric administration group: The drug concentration was prepared as 1 mg/mL, and the preparation solvent was Tween80 + 0.5% MC (v/v 1:99).
Drug preparation for the intravenous administration group: The drug concentration was prepared as 0.2 mg/mL, and the preparation solvent was 5% DMA + 5% Solutol HS-15 + 90% normal saline.

2.4. Administration:

Male SPF-grade KM mice were adaptively fed for 3-4 days.

Intragastric administration group: The administration dose was 10 mg/kg, and the administration volume was 10 mL/kg.

Intravenous administration group: The administration dose was 1 mg/kg, and the administration volume was 5 mL/kg.

2.5. Sample collection

Intragastric administration: Blood was collected from mice before administration (0 h) and after administration at 0, 0.25 h, 0.5 h, 1 h, 2 h, 4 h, 6 h, 8 h, 10 h, and 24 h. Intravenous injection: Blood was collected before administration (0 h) and after administration at 5, 15, 30 min, 1, 2, 4, 6, 8, and 24 h.

Blood, approximately 0.1 mL, was collected by retro-orbital puncture and placed into EDTA-K2 anticoagulant tubes with labels. After blood collection, the tubes were gently and completely inverted three times to mix with the anticoagulant. The samples were then immediately placed in an ice-water bath and centrifuged at 4500 rpm for 10 min at 4°C. After centrifugation, the plasma was promptly aliquoted into EP tubes with corresponding labels and stored in a -80°C freezer.

2.6. Sample testing

After administration, 20 μL of mouse plasma was taken and placed into a 96-deep-well plate pre-filled with 20 μL of internal standard working solution. Methanol (400 μL) as the precipitating agent was added, vortexed for 10 min, and centrifuged at 4000 rpm for 15 min. A supernatant of 200 μL was transferred into another 96-deep-well plate pre-filled with 200 μL of ultrapure water, vortexed for 5 min, and centrifuged at 4000 rpm for 3 min. A supernatant of 5 μL was taken for LC-MS/MS analysis of plasma drug concentration.

3. Experimental results and analysis

The main pharmacokinetic parameters were calculated by WinNonlin 7.0, and the pharmacokinetic experimental results of mice are shown in Table 5 below.

Table 5: Pharmacokinetic experimental results of mice

| No. | Administration method/ administration dose (mg/kg) | Half-life | Time to peak | Peak concentration | Area under the curve | Clearance | Apparent distribution (mL/kg) | Bioavailability |
|---|---|---|---|---|---|---|---|---|
| | | $t_{1/2}$ (h) | $T_{max}$ (h) | $C_{max}$/C0 (ng/mL) | $AUC_{0-t}$ (h × ng/mL) | CL/F (mL/h/kg) | Vss | % |
| Reference compound | i.g./10 | 1.97 | 1 | 1329.67 | 4526.66 | / | / | 44.2 |
| | i.v./1 | 1.99 | / | 621.18 | 1024.63 | 913.33 | 2482.96 | |
| Example 11 | i.g./10 | 1.37 | 0.25 | 3413.33 | 7537.94 | / | / | 71.4 |
| | i.v./1 | 1.44 | / | 612.43 | 1055.15 | 936.55 | 1399.24 | |

4. Experimental conclusion

It was known from the above data that, under the same administration dose, the exposure of the compound of the present disclosure was higher than that of the reference compound, and the bioavailability was also higher, indicating that the compound of the present disclosure exhibited better absorption characteristics.

**Test Example 6: Efficacy test in a mouse sodium acetate model**

1. Objective: The analgesic efficacy of the example was evaluated in a KM mouse acetic acid-induced writhing model.
2. Experimental methods:

2.1 Experimental drugs: The compounds in examples of the present disclosure and reference compound were self-prepared. Naproxen, Shanghai Yuanye Bio-Technology Co., Ltd., Catalog No. S63435. It was prepared using a solvent (Tween 80 + 0.5% MC (v:v = 1:99 pH = 3)).
2.2 Experimental animals: Male KM mice were purchased from Hubei Provincial Center for Disease Control and Prevention (Hubei Provincial Academy of Preventive Medicine), weighing 20 to 25 g at the time of purchase.
2.3 Experimental grouping:

Table 6: Efficacy test grouping of compounds in the acetic acid-induced writhing model

| Group No. | Group | Number of animals (n) | Administration dose (mg/kg) | Administration method (0.1 mL/10 g) | Modeling time |
|---|---|---|---|---|---|
| 1 | Solvent control group (Tween 80 + 0.5% MC (v:v = 1:99)) | 10 | / | Intragastric administration | 60 min after administration |
| 2 | Positive drug group (Naproxen) | 10 | 100 | Intragastric administration | 60 min after administration |
| 3 | Example 10 | 10 | 30 mg/kg | Intragastric administration | 60 min after administration |
| 4 | Solvent control group | 10 | / | Intragastric administration | 60 min after administration |
| 5 | Positive drug group (Naproxen) | 10 | 100 mg/kg | Intragastric administration | 60 min after administration |
| 6 | Reference compound | 10 | 30 mg/kg | Intragastric administration | 60 min after administration |
| 7 | Reference compound | 10 | 100 mg/kg | Intragastric administration | 60 min after administration |
| 8 | Example 11 | 10 | 30 mg/kg | Intragastric administration | 30 min after administration |
| 9 | Example 11 | 10 | 100 mg/kg | Intragastric administration | 30 min after administration |

**2.4 Administration and modeling**

One hour after the completion of the administration of the corresponding drugs in each group, 0.6% acetic acid (0.15 mL/10 g (0.1 mL/10 g also possible)) was injected intraperitoneally, the writhing latency (the time when mice first exhibited a writhing response after the injection of glacial acetic acid) was recorded, and the number of writhing occurrences within 20 min in mice was observed and recorded.
·Writhing index: Mice exhibited a typical abdominal concavity, accompanied by characteristic reactions such as torsion of the trunk and elevation of the hips, indicating the occurrence of writhing.

**2.5 Data collection and analysis**

Data were collected using Excel software.
Data were analyzed using Prism (Graph pad software, Inc.) software.

3. Results

Table 7: Analgesic efficacy results of compounds in the mouse acetic acid-induced writhing pain model

| Group | Administration method | Administration dose | Number of writhes | p (vs solvent control) | Number of animals |
|---|---|---|---|---|---|
| Solvent control group | Intragastric administration, single dose | 0.1 mL/10 g | $16.11 \pm 5.84$ | / | 10 |
| Positive drug group (Naproxen) | Intragastric administration, single dose | 100 mg/kg | $8.50 \pm 4.50$ | < 0.05 | 10 |
| Example 10 | Intragastric administration, single dose | 30 mg/kg | $12.10 \pm 6.10$ | < 0.05 | 10 |
| Solvent control group | Intragastric administration, single dose | 0.1 mL/10 g | $20.11 \pm 3.55$ | / | 10 |
| Positive drug group (Naproxen) | Intragastric administration, single dose | 100 mg/kg | $5.86 \pm 3.53$* | < 0.05 | 10 |
| Reference compound | Intragastric administration, single dose | 30 mg/kg | $11.86 \pm 3.53$* | < 0.05 | 10 |
| Reference compound | Intragastric administration, single dose | 100 mg/kg | $10.13 \pm 3.64$* | < 0.05 | 10 |
| Example 11 | Intragastric administration, single dose | 30 mg/kg | $11.56 \pm 5.15$* | < 0.05 | 10 |
| Example 11 | Intragastric administration, single dose | 100 mg/kg | $8.38 \pm 6.12$* | < 0.05 | 10 |

4. Conclusion

It was known from the above data that, at the same administration dose, the compounds of Example 10 and Example 11 of the present disclosure were able to inhibit acetic acid-induced pain in mice, reduce the number of writhes in mice, and Example 11 showed an obvious dose dependence in analgesic effect, with efficacy stronger than the reference compound.

## Claims

**1.** A compound of Formula I or a pharmaceutically acceptable salt thereof,

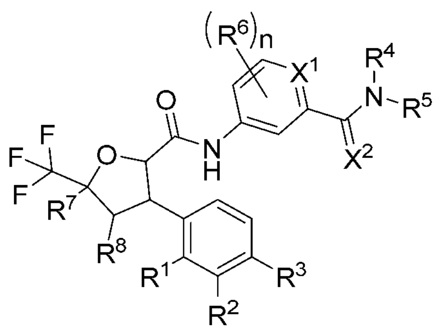

I ;

wherein $R^1$, $R^2$, and $R^3$ are each independently halogen, $C_1$-$C_6$ alkyl, $C_3$-$C_6$ cycloalkyl, $C_1$-$C_6$ alkoxy, $-OR^{1-1}$, $C_1$-$C_6$ alkyl substituted with one or more $R^{1-2}$, or $C_1$-$C_6$ alkoxy substituted with one or more $R^{1-3}$;
$R^{1-1}$ is $C_3$-$C_6$ cycloalkyl or 3- to 8-membered heterocycloalkyl; in the 3- to 8-membered heterocycloalkyl, the heteroatom is selected from 1, 2, or 3 types of N, O, and S, and the number of heteroatoms is 1, 2, or 3;
each $R^{1-2}$ is independently halogen;

each $R^{1-3}$ is independently $C_1$-$C_6$ alkoxy;
$X^1$ is N, $N^+$-$O^-$, or $CR^{X1}$;
$R^{X1}$ is hydrogen, halogen, or $C_1$-$C_6$ alkyl substituted with one or more halogens;
$X^2$ is O or NH;
$R^4$ is hydrogen or $C_1$-$C_6$ alkyl;
$R^5$ is $C_1$-$C_6$ alkoxy, -OH, -$NR^{5-1}R^{5-2}$, or -CN;
$R^{5-1}$ and $R^{5-2}$ are each independently hydrogen or $C_1$-$C_6$ alkyl;
n is 0, 1, 2, or 3;
$R^6$ is hydrogen, halogen, hydroxyl, $C_1$-$C_6$ alkyl, $C_1$-$C_6$ alkyl-$C_1$-$C_6$ alkoxy, or $C_1$-$C_6$ alkoxy;
$R^7$ is hydrogen or $C_1$-$C_6$ alkyl;
$R^3$ is hydrogen, hydroxyl, $C_1$-$C_6$ alkyl, $C_1$-$C_6$ alkyl-$C_1$-$C_6$ alkoxy, or $C_1$-$C_6$ alkoxy.

**2.** The compound of Formula I or the pharmaceutically acceptable salt thereof according to claim 1, wherein the compound of Formula I or the pharmaceutically acceptable salt thereof satisfies one or more of the following conditions:

(1) in $R^1$, $R^2$, and $R^3$, each halogen is independently fluorine, chlorine, bromine, or iodine, preferably fluorine;
(2) in $R^1$, $R^2$, and $R^3$, each $C_1$-$C_6$ alkyl in the $C_1$-$C_6$ alkyl and the $C_1$-$C_6$ alkyl substituted with one or more $R^{1-2}$ is independently methyl, ethyl, n-propyl, isopropyl, n-butyl, isobutyl, or *tert-butyl,* preferably methyl;
(3) in $R^1$, $R^2$, and $R^3$, each $C_3$-$C_6$ cycloalkyl is independently cyclopropyl, cyclobutyl, cyclopentyl, or cyclohexyl, preferably cyclopropyl;
(4) in $R^1$, $R^2$, and $R^3$, each $C_1$-$C_6$ alkoxy in the $C_1$-$C_6$ alkoxy and the $C_1$-$C_6$ alkoxy substituted with one or more $R^{1-3}$ is independently methoxy, ethoxy, n-propoxy, isopropoxy, n-butoxy, isobutoxy, or tert-butoxy, preferably methoxy or ethoxy;
(5) in $R^{1-1}$, the $C_3$-$C_6$ cycloalkyl is cyclopropyl, cyclobutyl, cyclopentyl, or cyclohexyl, preferably cyclopropyl;
(6) in $R^{1-1}$, the heteroatom in the 3- to 8-membered heterocycloalkyl is N and/or O; the number of heteroatoms is preferably 1 or 2; the 3- to 8-membered heterocycloalkyl is preferably 4- to 6-membered heterocycloalkyl; more preferably

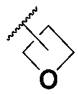 ;

for example,

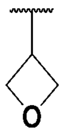 ;

(7) in $R^{1-2}$, the halogen is fluorine, chlorine, bromine, or iodine, preferably fluorine;
(8) in $R^{1-3}$, the $C_1$-$C_6$ alkoxy is methoxy, ethoxy, n-propoxy, isopropoxy, n-butoxy, isobutoxy, or tert-butoxy, preferably methoxy;
(9) in $R^{X1}$, each halogen in the halogen and the $C_1$-$C_6$ alkyl substituted with one or more halogens is independently fluorine, chlorine, bromine, or iodine, preferably fluorine;
(10) in $R^{X1}$, the $C_1$-$C_6$ alkyl in the $C_1$-$C_6$ alkyl substituted with one or more halogens is methyl, ethyl, n-propyl, isopropyl, n-butyl, isobutyl, or *tert-butyl,* preferably methyl;
(11) in $R^5$, the $C_1$-$C_6$ alkoxy is methoxy, ethoxy, n-propoxy, isopropoxy, n-butoxy, isobutoxy, or tert-butoxy, preferably methoxy or ethoxy;
(12) in $R^{5-1}$ and $R^{5-2}$, each $C_1$-$C_6$ alkyl is independently methyl, ethyl, n-propyl, isopropyl, n-butyl, isobutyl, or *tert-butyl,* preferably methyl;
(13) in $R^6$, the halogen is fluorine, chlorine, bromine, or iodine, preferably fluorine;
(14) in $R^7$, the $C_1$-$C_6$ alkyl is methyl, ethyl, n-propyl, isopropyl, n-butyl, isobutyl, or *tert*-butyl, preferably methyl; and
(15) in $R^8$, the $C_1$-$C_6$ alkyl is methyl, ethyl, n-propyl, isopropyl, n-butyl, isobutyl, or *tert*-butyl, preferably methyl.

**3.** The compound of Formula I or the pharmaceutically acceptable salt thereof according to claim 1, wherein the

compound of Formula I or the pharmaceutically acceptable salt thereof satisfies one or more of the following conditions:

(1) $R^1$ is $C_1$-$C_6$ alkyl, $C_3$-$C_6$ cycloalkyl, $C_1$-$C_6$ alkoxy, $-OR^{1-1}$, $C_1$-$C_6$ alkyl substituted with one or more $R^{1-2}$, or $C_1$-$C_6$ alkoxy substituted with one or more $R^{1-3}$;
(2) $R^2$ is halogen;
(3) $R^3$ is halogen;
(4) $R^4$ is hydrogen;
(5) $R^{5-1}$ is hydrogen;
(6) $R^{5-2}$ is hydrogen or $C_1$-$C_6$ alkyl;
(7) $R^6$ is halogen;
(8) $R^7$ is $C_1$-$C_6$ alkyl; and
(9) $R^8$ is $C_1$-$C_6$ alkyl.

**4.** The compound of Formula I or the pharmaceutically acceptable salt thereof according to claim 1, wherein the compound of Formula I or the pharmaceutically acceptable salt thereof is a compound of Formula I-1 or a pharmaceutically acceptable salt thereof:

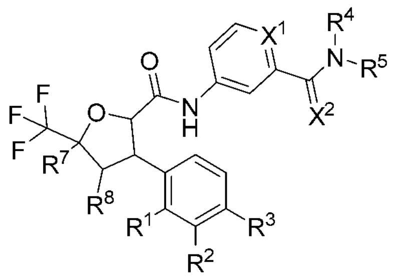

I-1 ;

$R^1$ is $C_1$-$C_6$ alkoxy or $C_1$-$C_6$ alkoxy substituted with one or more $R^{1-3}$;
each $R^{1-3}$ is independently $C_1$-$C_6$ alkoxy;
$R^2$ is halogen;
$R^3$ is halogen;
$X^1$ is N or $CR^{X1}$;
$R^{X1}$ is halogen;
$X^2$ is O or NH;
$R^4$ is hydrogen;
$R^5$ is -OH or -CN;
$R^7$ is $C_1$-$C_6$ alkyl;
$R^8$ is $C_1$-$C_6$ alkyl.

**5.** The compound of Formula I or the pharmaceutically acceptable salt thereof according to claim 3, wherein the compound of Formula I or the pharmaceutically acceptable salt thereof satisfies one or more of the following conditions:

(1) $R^1$ is methyl, cyclopropyl, methoxy, $-CF_3$,

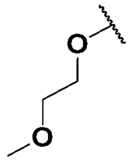 , 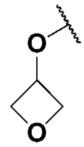 , or 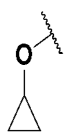 ;

(2) $R^2$ is fluorine;
(3) $R^3$ is fluorine;
(4) $X^1$ is N, $N^+$-$O^-$, CH, CF, or C-$CF_3$; and

(5) $R^5$ is methoxy, ethoxy, -OH, $-NH_2$, $-NH(CH_3)$, or -CN.

**6.** The compound of Formula I or the pharmaceutically acceptable salt thereof according to claim 5, wherein

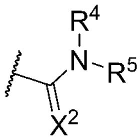

is

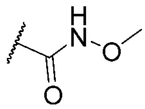, 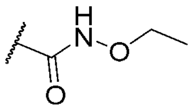, 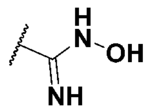, 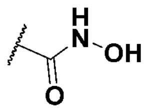,

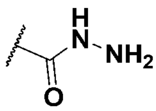, 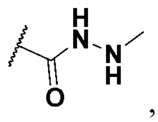, or 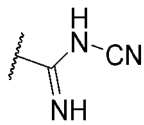.

**7.** The compound of Formula I or the pharmaceutically acceptable salt thereof according to any one of claims 1-6, wherein the compound of Formula I is any one of the following compounds:

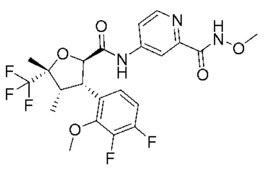

BX20-9-021

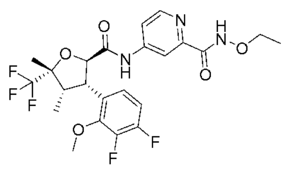

BX20-9-022

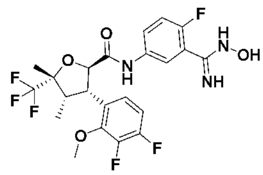

BX20-9-029

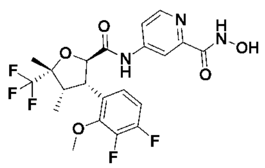

BX20-9-033

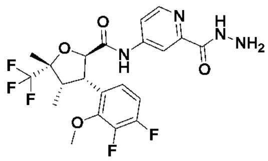

BX20-9-034

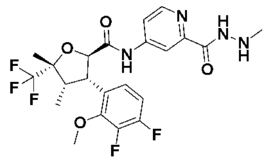

BX20-9-035

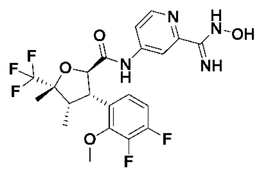

BX20-9-036

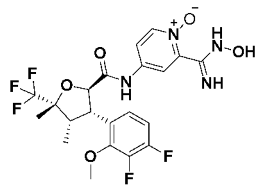

BX20-9-037

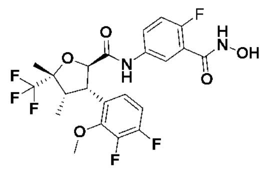

BX20-9-038

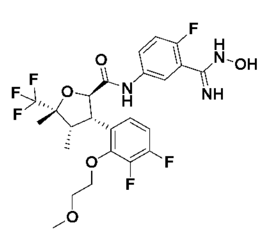

BX20-9-039

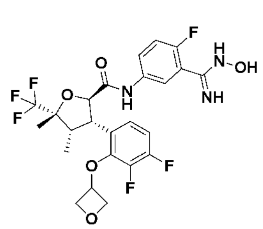

BX20-9-040

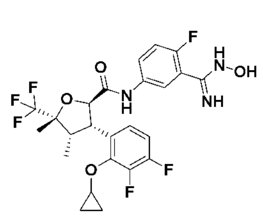

BX20-9-041

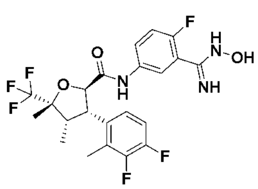

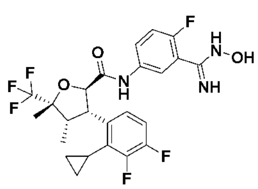

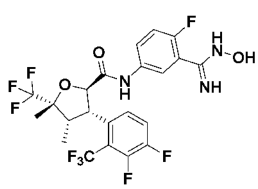

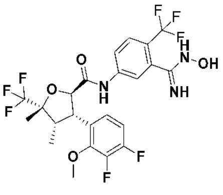
BX20-9-046

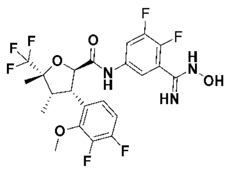
BX20-9-047

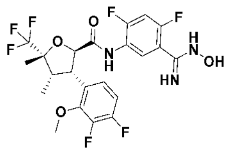
BX20-9-048

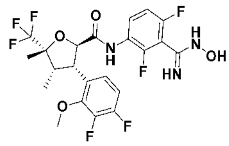
BX20-9-049

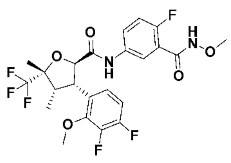
BX20-9-052

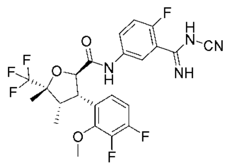
BX20-9-055

**8.** A pharmaceutical composition, wherein the pharmaceutical composition comprises:

(1) the compound of Formula I or the pharmaceutically acceptable salt thereof according to any one of claims 1-7, and
(2) a pharmaceutically acceptable excipient.

**9.** A use of substance A in the manufacture of a medicament for treating a disease; wherein the disease may be pain, a pain-related disease, multiple sclerosis, incontinence, or arrhythmia; the pain is preferably one or more of acute pain, chronic pain, inflammatory pain, cancer pain, neuropathic pain, musculoskeletal pain, primary pain, intestinal pain, or idiopathic pain;

alternatively, the disease may be a disease treatable by inhibiting voltage-gated sodium channels; the voltage-gated sodium channel is preferably $Na_V1.8$;
the substance A is the compound of Formula I or the pharmaceutically acceptable salt thereof according to any one of claims 1-7, or the pharmaceutical composition according to claim 8.

**10.** A use of substance A in the manufacture of a voltage-gated sodium channel inhibitor; wherein the voltage-gated sodium channel is preferably $Na_V1.8$;
the substance A is as described in claim 9.

**11.** A use of substance A in the manufacture of a medicament for a disease caused by abnormal activation of voltage-gated sodium channels; wherein the voltage-gated sodium channel is preferably $Na_V1.8$;

the disease may be pain, a pain-related disease, multiple sclerosis, incontinence, or arrhythmia;
the substance A is as described in claim 9.

**INTERNATIONAL SEARCH REPORT**

International application No.
**PCT/CN2024/133203**

**A. CLASSIFICATION OF SUBJECT MATTER**

C07D 405/12(2006.01)i; C07D 405/14(2006.01)i; C07D 413/14(2006.01)i; C07D 471/04(2006.01)i; C07D 471/08(2006.01)i; C07D 487/10(2006.01)i; C07D 491/048(2006.01)i; C07D 491/08(2006.01)i; C07D 491/10(2006.01)i; C07D 493/10(2006.01)i; C07D 498/04(2006.01)i; C07D 498/08(2006.01)i; A61K 31/443(2006.01)i; A61K 31/497(2006.01)i; A61P 29/00(2006.01)i

According to International Patent Classification (IPC) or to both national classification and IPC

**B. FIELDS SEARCHED**

Minimum documentation searched (classification system followed by classification symbols)

IPC: C07D 405, C07D 413, C07D 471, C07D 487, C07D 491, C07D 493, C07D 498, A61K 31, A61P 29

Documentation searched other than minimum documentation to the extent that such documents are included in the fields searched

Electronic data base consulted during the international search (name of data base and, where practicable, search terms used)

REGISTRY/CAPLUS (STN), CNTXT, ENTXTC, ENTXT, DWPI, CNKI: 四氢呋喃, 酰胺, 苯基, 吡啶, 钠通道, Nav, 疼痛, tetrahydrofuran, amide, phenyl, pyridin+, sodium channel, pain, 根据权利要求1中化合物的结构检索, structural search according to compounds in claim 1

**C. DOCUMENTS CONSIDERED TO BE RELEVANT**

| Category* | Citation of document, with indication, where appropriate, of the relevant passages | Relevant to claim No. |
|---|---|---|
| PX | CN 118812519 A (WUHAN HUMANWELL INNOVATIVE DRUG R & D CENTER CO., LTD. et al.) 22 October 2024 (2024-10-22)<br>entire document, in particular abstract, the claims, description, compounds I-26 and I-28 to I-30 and I-40, description, paragraph 552, the compounds, and description, test example 1 | 1-11 |
| PX | CN 118388466 A (ARROMAX PHARMACEUTICAL TECHNOLOGY (SUZHOU) CO., LTD.) 26 July 2024 (2024-07-26)<br>entire document, in particular abstract, the claims, description, compounds I-7, 7F1, 7F2, I-8, 8F1, 8F2, I-10, 10F1 and 10F2, and description, test examples 1 and 2 | 1-11 |
| PX | WO 2024046253 A1 (SHANGHAI HUILUN PHARMACEUTICAL CO., LTD.) 07 March 2024 (2024-03-07)<br>entire document, in particular abstract, the claims, description, compounds 20 and 21, description, embodiments 20 and 21, and description, biological activity tests 1-3 | 1-11 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 January 2025** | **17 February 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2024/133203**

**C. DOCUMENTS CONSIDERED TO BE RELEVANT**

| Category* | Citation of document, with indication, where appropriate, of the relevant passages | Relevant to claim No. |
|---|---|---|
| PX | WO 2024041613 A1 (JIANGSU HENGRUI PHARMACEUTICALS CO., LTD. et al.) 29 February 2024 (2024-02-29)<br>entire document, in particular abstract, the claims, description, compounds 5, 12, 16, 19, 22, 24, 32, 34-37, 43 and 44, and description, test examples 1 and 4 | 1-11 |
| X | WO 2022256676 A1 (VERTEX PHARMACEUTICALS, INC.) 08 December 2022 (2022-12-08)<br>entire document, in particular abstract, the claims, description, page 76, table 3, second compound, description, paragraphs 120 and 174-197, description, page 302, compound 217, and description, embodiment 30 | 1-11 |

Form PCT/ISA/210 (second sheet) (July 2022)

**INTERNATIONAL SEARCH REPORT**
**Information on patent family members**

International application No.
**PCT/CN2024/133203**

| Patent document cited in search report | | | Publication date (day/month/year) | Patent family member(s) | | | Publication date (day/month/year) |
|---|---|---|---|---|---|---|---|
| CN | 118812519 | A | 22 October 2024 | WO | 2024217557 | A1 | 24 October 2024 |
| CN | 118388466 | A | 26 July 2024 | None | | | |
| WO | 2024046253 | A1 | 07 March 2024 | None | | | |
| WO | 2024041613 | A1 | 29 February 2024 | TW | 202409017 | A | 01 March 2024 |
| WO | 2022256676 | A1 | 08 December 2022 | EP | 4347583 | A1 | 10 April 2024 |
| | | | | AU | 2022285758 | A1 | 30 November 2023 |
| | | | | CA | 3221938 | A1 | 08 December 2022 |
| | | | | JP | 2024522293 | A | 13 June 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023115572534
- CN 2024100374874
- CN 114945566 A


### Non-patent literature cited in the description


- **P. HEINRICH STAHL** ; **CAMILLE G. WERMUTH**. Handbook of Pharmaceutical Salts: Properties, Selection, and Use. 2011
- Pharmacopoeia of the People's Republic of China. 2020
- **PAUL J SHESKEY** ; **BRUNO C HANCOCK** ; **GARY P MOSS** ; **DAVID J GOLDFARB**. *Handbook of Pharmaceutical Excipients*, 2020
- *Proc Natl Acad Sci U S A.*, 26 July 2022, vol. 119 (30), e2208211119
- **JARVIS MF** ; **HONORE P** ; **SHIEH CC** ; **CHAPMAN M** ; **JOSHI S** ; **ZHANG XF** ; **KORT M** ; **CARROLL W** ; **MARRON B** ; **ATKINSON R**. Krafte DS. A-803467, a potent and selective Nav1.8 sodium channel blocker, attenuates neuropathic and inflammatory pain in the rat. *Proc Natl Acad Sci U S A.*, 02 May 2007, vol. 104 (20), 8520-5